# EUROPEAN PATENT SPECIFICATION

(11) **EP 1 328 254 B1**
(45) Date of publication and mention of the grant of the patent: **21.02.2007**
(21) Application number: 01983342.5
(22) Date of filing: 25.10.2001
(51) Int. Cl.: A61K 9/127

(54) **LIPID FORMULATIONS FOR TARGET DELIVERY**
LIPIDFORMULIERUNGEN ZUR ZIELGERICHTETEN ABGABE
FORMULATIONS DE LIPIDES POUR UNE ADMINISTRATION CIBLEE

(30) Priority: 25.10.2000 US 243185 P
(43) Date of publication of application: 23.07.2003
(73) Proprietor: THE UNIVERSITY OF BRITISH COLUMBIA, Vancouver, British Columbia V6T 1Z3 (CA); INEX Pharmaceuticals Corp., Burnaby, British Columbia V6K 3S4 (CA)
(72) Inventor: CULLIS, Pieter, R., Vancouver, British Columbia V6R 1H4 (CA); MACLACHLAN, Ian, Vancouver, British Columbia V5T 4S2 (CA); FENSKE, David, B., Surrey, British Columbia V3S 7L1 (CA)
(74) Representative: Manitz, Finsterwald & Partner GbR
(86) International application number: PCT/CA2001/001513
(87) International publication number: WO 2002/034236

(56) References cited:
- WO-A-00/62813
- WO-A-96/10392
- WO-A-98/51278
- WO-A-99/04819

## Description

### FIELD OF THE INVENTION

This invention relates to lipid formulations, and more particularly, to nucleic acid-lipid particles useful as targeted gene delivery vehicles.

### BACKGROUND OF THE INVENTION

Current gene delivery systems for systemic gene therapy include viral and non-viral systems. Viral gene transfer systems are rapidly cleared from circulation upon systemic administration, limiting their use to first pass organs such as the liver or lungs. In addition, the immunogenicity of viral systems directs an acute inflammatory response, which at best limits the effect of subsequent doses and at worst can be hazardous to the patient (*see,* Worgall *et al*., *Human Gene Therapy,* **8:** 37-44,1997). Non-viral systems, such as plasmid-DNA-cationic lipid complexes (lipoplexs) perform adequately *in vitro,* but due to their large size and positive surface charge, lipoplex are also rapidly cleared from circulation and are similarly limited to applications involving transfection of first pass organs *(see,* Hofland *et al.*, *Pharmaceutical Res*, **14**: 742-749 1997). DNA-cationic lipid complexes have also been shown to exhibit significant toxicity *(see,* Li and Huang, *Gene Therapy,* **4:** 891-900, 1997).

A fundamental hurdle for gene drugs is delivery of the gene to an area of interest because large nucleic acids are rapidly degraded upon exposure to serum. However, this hurdle has been overcome for local and regional (*i.e*., inhalation or direct injection) delivery by the use of viral vectors, lipid complexes and the like. For systemic (*i.e.*, intravenous) delivery, the hurdle has been overcome by the use of stable plasmid-lipid particles ("SPLPs") disclosed in PCT Publication No. WO 96/40964, which is incorporated herein by reference. SPLPs are fully encapsulated lipid-plasmid particles that are resistant to nuclease degradation, have low immunogenicity, and are of small size (<150 nm), thereby making them particularly well suited for long circulation lifetimes.

Various SPLP compositions have been disclosed that improve circulation-characteristics and serum stability (*see e*.*g*. WO 9.6/10392 disclosing a SPLP containing a cationic lipid, phophatidylethanolamine and a bilayer stabilizing component; WO 99/04819 disclosing a SPLP containing a nucleic acid, a cationic lipid and a PEG-ceramide conjugate) that improve encapsulation efficiency when using charged therapeutic agents (*see* WO 98/31278 disclosing a SPLP containing a nucleic acid, a protonatable amino lipid and a PEG-modified lipid) and that improve cellular uptake (WO 00/62813 disclosing a SPLP using a conjugate of a lipid moiety and a hydrophilic polymer with a cationic moiety).

Despite the advances made in SPLP technology, none of these technologies have provided a method for identifying a lipid-based systemic delivery vehicle capable of selectively targeting specific tissue sites, by varying the amount of each constituent part of the vehicle to impart tissue selectivity for a specific tissue site as is presently disclosed in the present invention.

The SPLPs can be produced using detergent dialysis methods that allows for complete encapsulation of plasmid DNA inside small (~70 nm), well defined lipid vesicles (*see,* Wheeler *et al., Gene Therapy,* **6**: 271-281, 1999). These vesicles contain fusogenic helper lipids, which are known to induce fusion with cellular membranes. Since fusogenic lipids do not readily form the bilayer structured needed for liposome formation; they require stabilization or aggregating preventing agent such as polyethylene glycol (PEG)-lipid conjugates or anchors. The resulting particles have been shown to have circulation half-lives that are dependent on the length of the lipid anchor. Circulation half lives varied from 1.2 min (PEG-CerC₈) to 13 h (PEG-CerC₂₀). This property permits the construction of a 'programmable' fusogenic gene delivery system. As the PEG-Cer diffuses out of the SPLP membrane at a rate that is dependent on its anchor length, the SPLP becomes more unstable and fusogenic, resulting in membrane fusion and release of the plasmid DNA payload. These particles exhibit extended circulation lifetimes, and enhanced pharmacokinetics over viral and other non-viral systems. *In vitro* studies have shown SPLP's with high levels of cationic lipid (24 mol %) have transfection properties, which are better than plasmid DNA-cationic lipid complexes.

Despite the advances made in the SPLP technology, what is needed in the art are formulations that allow delivery of a payload to a target, *i*.*e*., a particular tissue site of interest. The present invention fulfills this and other needs.

### SUMMARY OF THE INVENTION

According to the present invention there is provided an *in vitro* method for identifying a nucleic acid-lipid particle capable of selectively targeting a nucleic acid to a heart, liver, or tumor tissue site, said method comprising:
designing a nucleic acid-lipid particle comprising:
   (a) a nucleic acid;
   (b) 1.0 mole % to 45 mole % of a cationic lipid;
   (c) 0,0 mole % to 90 mole % of another lipid;
   (d) 1,0 mole % to 10 mole % of a bilayer stabilizing component;
   (e) 0,0 mole % to 60 mole % cholesterol; and
   (f) 0,0 mole % to 10 mole % of cationic polymer lipid;
   and
varying the amount of each of said cationic lipid, bilayer stabilizing component, another lipid, cholesterol, and cationic polymer lipid to impart tissue selectivity for said heart, liver, or tumor tissue site, thereby identifying a nucleic acid-lipid particle capable of selectively targeting a nucleic acid to said heart, liver, or tumor tissue site.

Preferably, the nucleic acid is selected from the group consisting of a plasmid and antisense oligonucleotide and a ribozyme.

According to a preferred embodiment of the present patent application, the nucleic acid-lipid particle to be identified excludes targeting moieties.

Preferably, the nucleic acid-lipid particle comprises DOPE, DODAC and PEGC₂₀.

The ratio of DOPE:DODAC:PEGC₂₀ is preferably 76 mole % to 14 mole % to 10 mole %.

According to another embodiment of the present patent application, the nucleic acid-lipid particle comprises DOPE, CHOL, DODAC, PEGC₂₀ and a CPL, with the ratio of DOPE:CHOL:DODAC:PEGC₂₀:CPL being preferably 31 mole % to 45 mole % to 14 mole % to 10 mole % to 4 mole %.

In another embodiment of the present patent application, the ratio of DOPE:DODAC:PEGC₂₀ is 82,5 mole % to 7,5 mole % to 10 mole %.

### BRIEF DESCRIPTION OF THE DRAWINGS

**Figure 1** illustrates SPLP can be engineered to elicit tumor specific gene expression.
**Figure 2** illustrates SPLP for liver gene expression. As shown therein, the SPLPs are rapidly cleared from the blood compartment.
**Figure 3** illustrates SPLP for liver gene expression. These SPLPs have been designed for effective delivery to the liver.
**Figure 4** illustrates gene expression in subcutaneous Neuro2A tumors following LV. injection of SPLP.

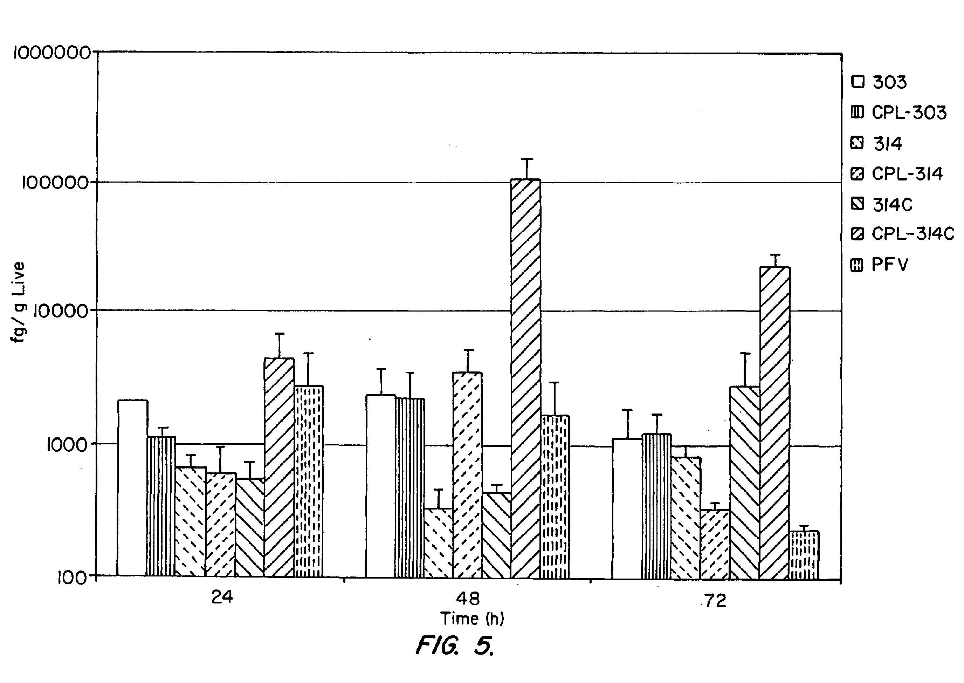

**Figure 5** illustrates luciferase gene expression in the liver from Neuro-2a tumor bearing male A/J mice.

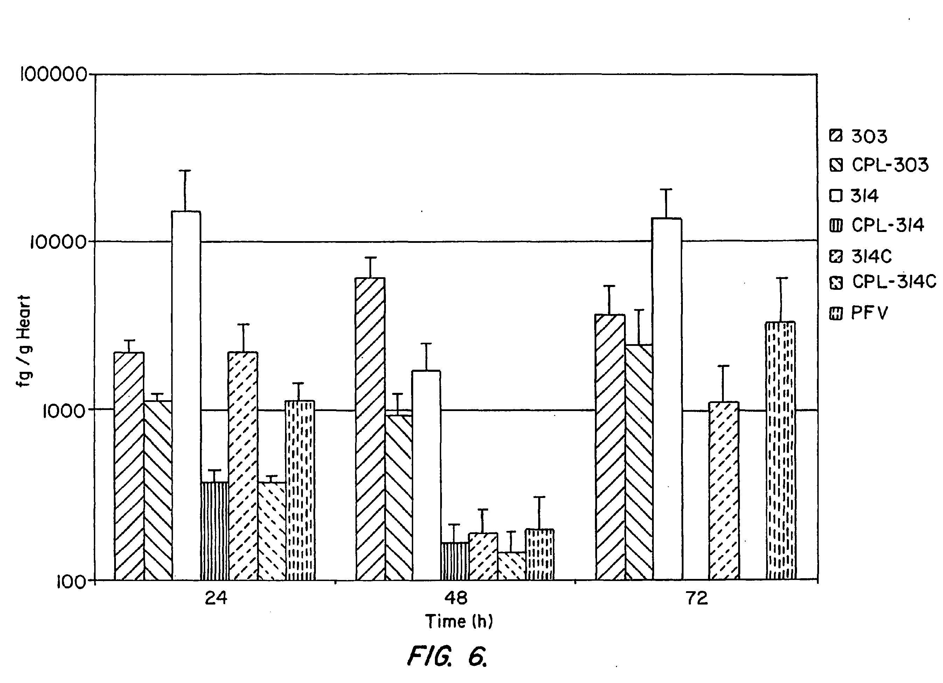

**Figure 6** illustrates luciferase gene expression in the heart from Neuro-2a tumor bearing male A/J mice.

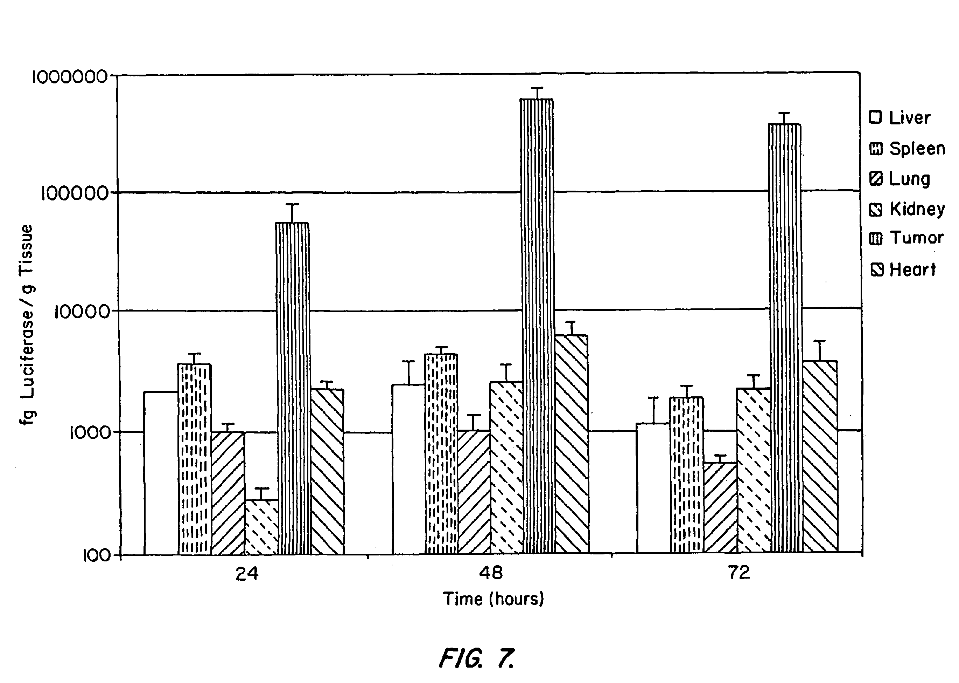

**Figure 7** illustrates luciferase gene expression in Neuro-2a tumor bearing mice following a single IV administration of INEX 303.

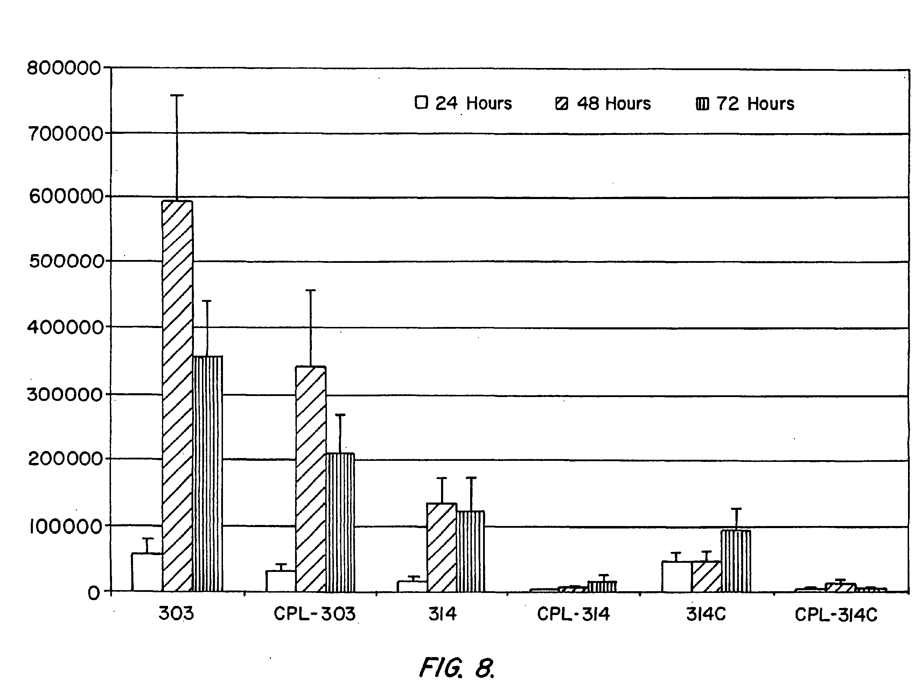

**Figure 8** illustrates potency of SPLP : tumor gene expression following intravenous administration of SPLP.

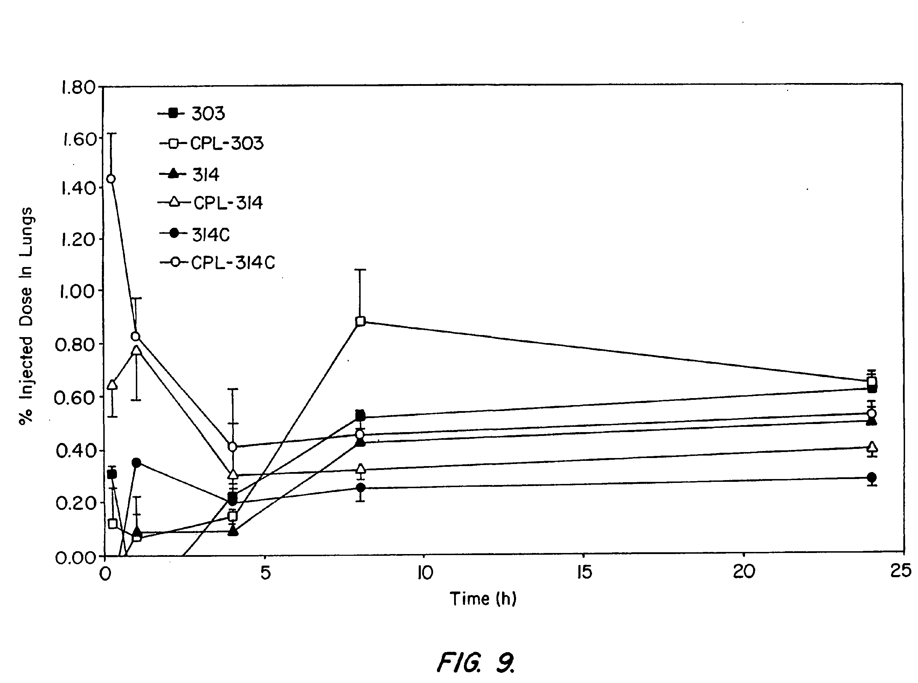

**Figure 9** illustrates percent injected dose in lungs of male A/J mice following a single intravenous administration of 303, 314, and 314C with or without 4 mol% CPL4-1k.

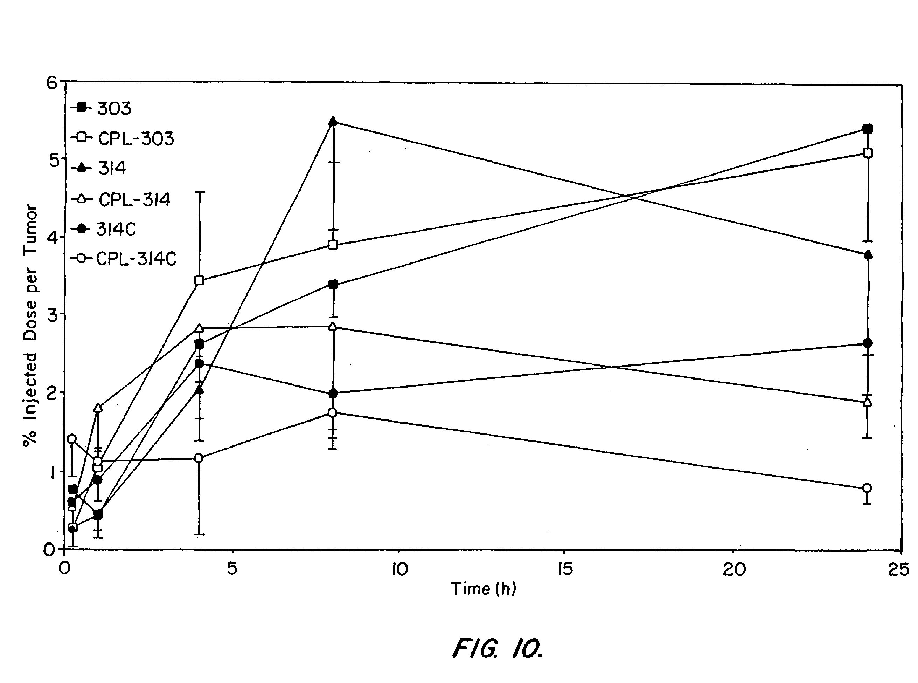

**Figure 10** illustrates percent injected dose per Neuro-2a tumor of male A/J mice following a single intravenous administration of 303, 314, and 314C with or without 4 mol% CPL4-1k.

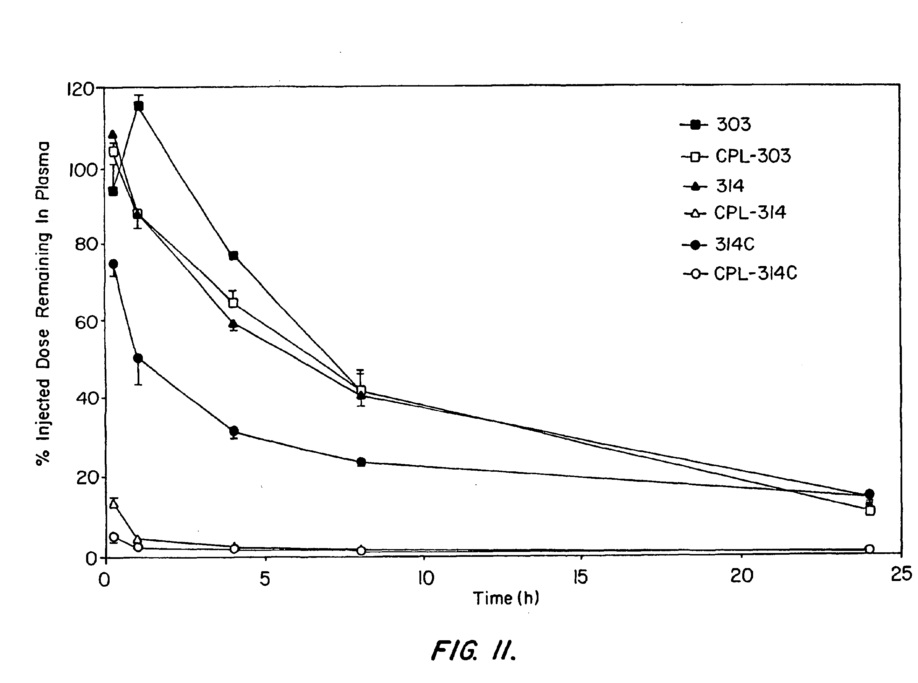

**Figure 11** illustrates percent injected dose remaining in plasma of male A/J mice following a single intravenous administration of 303, 314, and 314C with or without 4 mol % CPL4-1k.

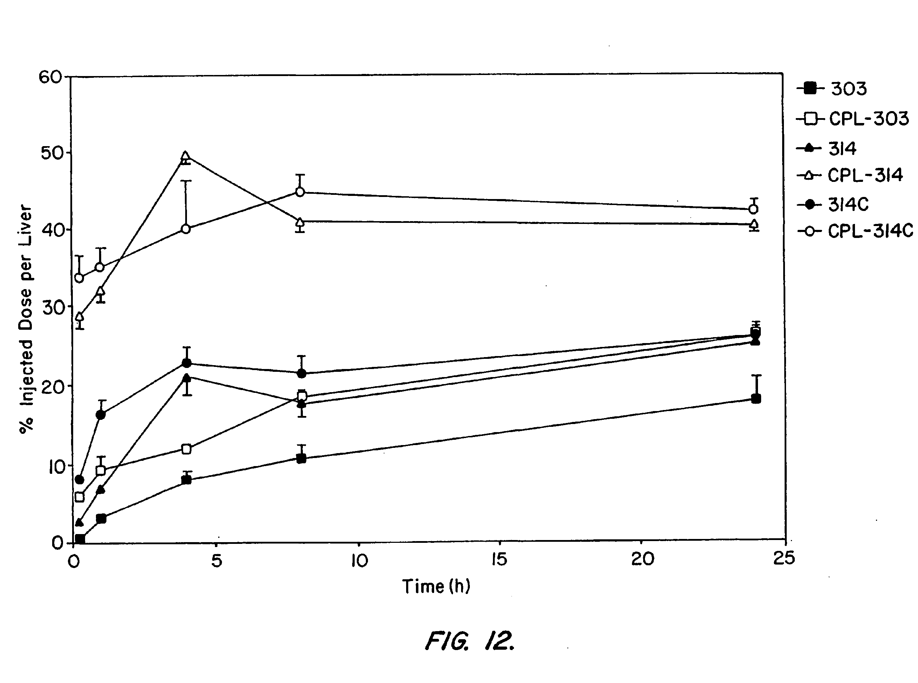

**Figure 12** illustrates percent dose per liver of male A/J mice following a single intravenous administration of 303,314, and 314C with or without 4 mol% CPL4-1k.

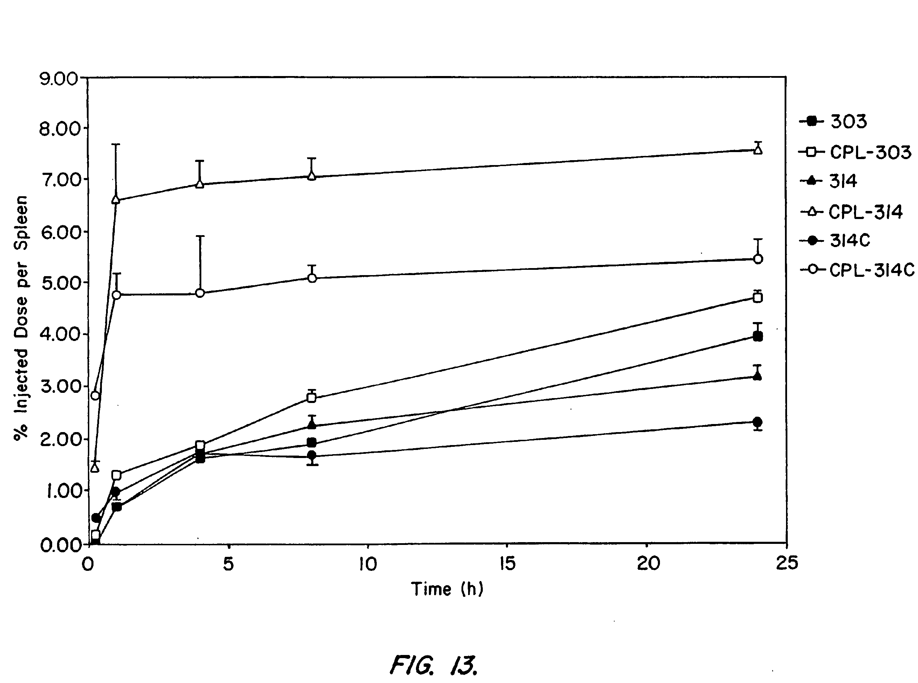

**Figure 13** illustrates percent injected dose per spleen of male A/J mice following a single intravenous administration of 303, 314, and 314C with or without 4 mol% CPL4-1k.

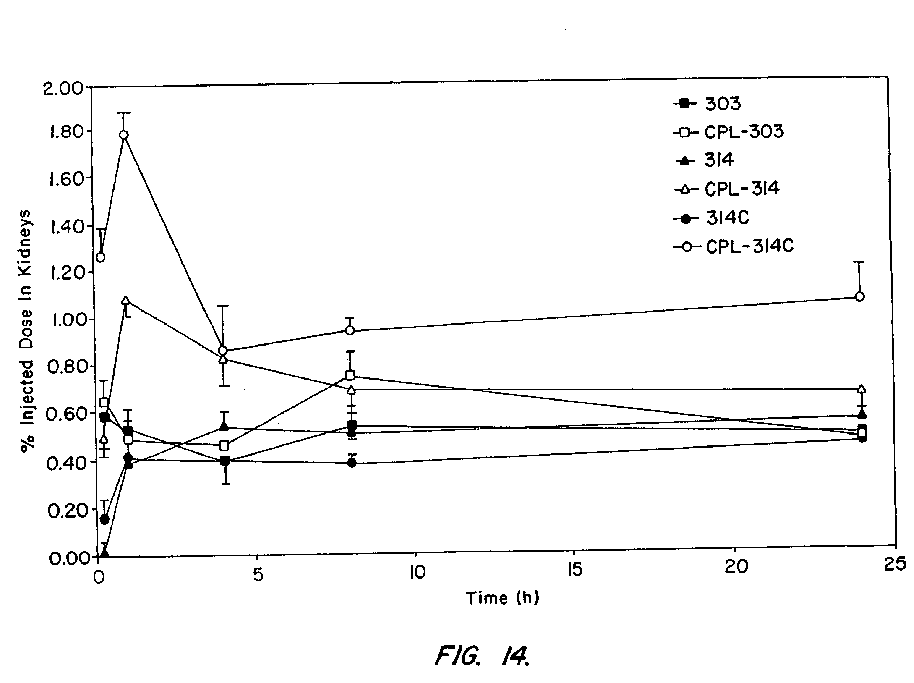

**Figure 14** illustrates percent injected dose in kidneys of male A/J mice following a single intravenous administration of 303, 314, and 314C with or without 4 mol% CPL4-1k.

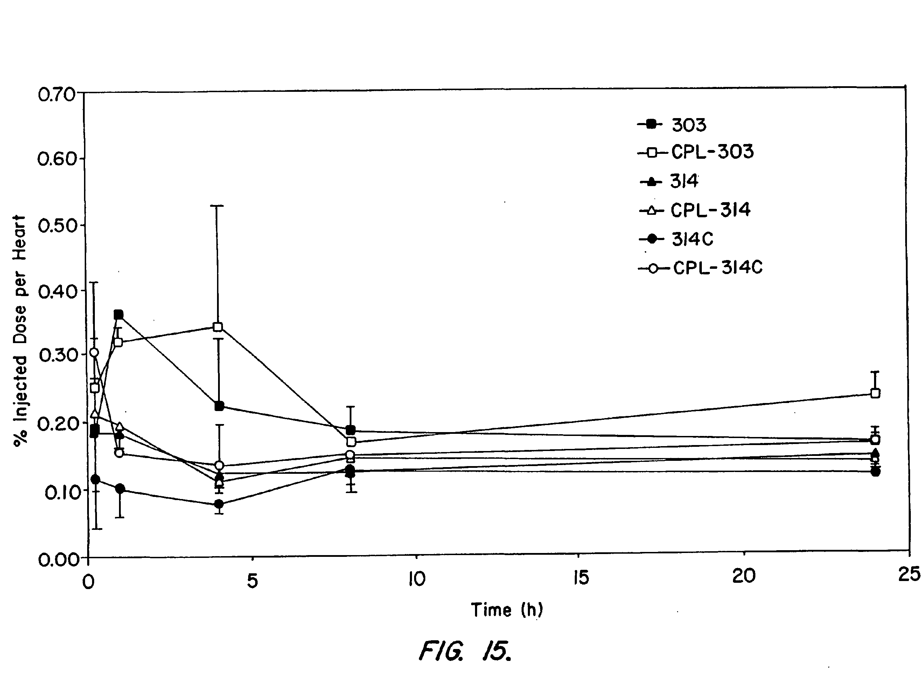

**Figure 15** illustrates percent injected dose in hearts of male A/J mice following a single intravenous administration of 303,314, and 314C with or without 4 mol% CPL4-1k.
**Figure 16** illustrates CPL chemistry.

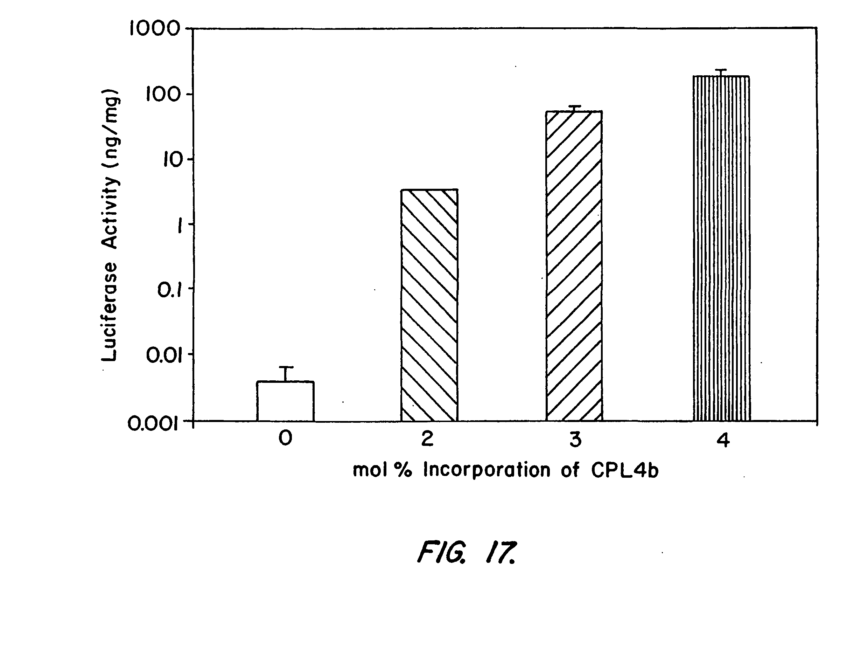

**Figure 17** illustrates CPL enhance *in vitro* transfection.

### DETAILED DESCRIPTION OF THE INVENTION AND PREFERRED EMBODIMENTS

### I. DEFINITIONS

The term "lipid" refers to a group of organic compounds that include, but are not limited to, esters of fatty acids and are characterized by being insoluble in water, but soluble in many organic solvents. They are usually divided into at least three classes: (1) "simple lipids" which include fats and oils as well as waxes; (2) "compound lipids" which include phospholipids and glycolipids; (3) "derived lipids" such as steroids.

The term "vesicle-forming lipid" is intended to include any amphipathic lipid having a hydrophobic moiety and a polar head group, and which by itself can form spontaneously into bilayer vesicles in water, as exemplified by most phospholipids.

The term "vesicle-adopting lipid" is intended to include any amphipathic lipid which is stably incorporated into lipid bilayers in combination with other amphipathic lipids, with its hydrophobic moiety in contact with the interior, hydrophobic region of the bilayer membrane, and its polar head group moiety oriented toward the exterior, polar surface of the membrane. Vesicle-adopting lipids include lipids that on their own tend to adopt a non-lamellar phase, yet which are capable of assuming a bilayer structure in the presence of a bilayer-stabilizing component. A typical example is DOPE (dioleoylphosphatidylethanolamine). Bilayer stabilizing components include, but are not limited to, polyamide oligomers, peptides, proteins, detergents, lipid-derivatives, PEG-lipid derivatives such as PEG coupled to phosphatidylethanolamines, and PEG conjugated to ceramides *(see,* U.S. Patent No. 5,885,613, which is incorporated herein by reference).

The term "amphipathic lipid" refers, in part, to any suitable material wherein the hydrophobic portion of the lipid material orients into a hydrophobic phase, while the hydrophilic portion orients toward the aqueous phase. Amphipathic lipids are usually the major component of a lipid vesicle. Hydrophilic characteristics derive from the presence of polar or charged groups such as carbohydrates, phosphato, carboxylic, sulfato, amino, sulfhydryl, nitro, hydroxy and other like groups. Hydrophobicity can be conferred by the inclusion of apolar groups that include, but are not limited to, long chain saturated and unsaturated aliphatic hydrocarbon groups and such groups substituted by one or more aromatic, cycloaliphatic or heterocyclic group(s). Examples of amphipathic compounds include, but are not limited to, phospholipids, aminolipids and sphingolipids. Representative examples of phospholipids include, but are not limited to, phosphatidylcholine, phosphatidylethanolamine, phosphatidylserine, phosphatidylinositol, phosphatidic acid, palmitoyloleoyl phosphatidylcholine, lysophosphatidylcholine, lysophosphatidylethanolamine, dipalmitoylphosphatidylcholine, dioleoylphosphatidylcholine, distearoylphosphatidylcholine or dilinoleoylphosphatidylcholine. Other compounds lacking in phosphorus, such as sphingolipid, glycosphingolipid families, diacylglycerols and β-acyloxyacids, are also within the group designated as amphipathic lipids. Additionally, the amphipathic lipid described above can be mixed with other lipids including triglycerides and sterols.

The term "neutral lipid" refers to any of a number of lipid species that exist either in an uncharged or neutral zwitterionic form at a selected pH. At physiological pH, such lipids include, for example, diacylphosphatidylcholine, diacylphosphatidylethanolamine, ceramide, sphingomyelin, cephalin, cholesterol, cerebrosides and diacylglycerols.

The term "hydrophopic lipid" refers to compounds having apolar groups that include, but are not limited to, long chain saturated and unsaturated aliphatic hydrocarbon groups and such groups optionally substituted by one or more aromatic, cycloaliphatic or heterocyclic group(s). Suitable examples include, but are not limited to, diacylglycerol, dialkylglycerol, N-N-dialkylamino, 1,2-diacyloxy-3-aminopiopane and 1,2-dialkyl-3-aminopropane.

The term "diacylglycerolyl" denotes 2-fatty acyl chains, R¹ and R² having independently between 2 and 30 carbons bonded to the 1- and 2-position of glycerol by ester linkages. The acyl groups can be saturated or have varying degrees of unsaturation. Diacylglycerol groups have the following general formula:

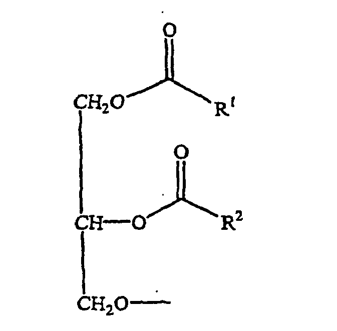

The term "dialkylglycerolyl" denotes two C₁-C₃₀ alkyl chains bonded to the 1-and 2-position of glycerol by ether linkages. Dialkylglycerol groups have the following general formula:

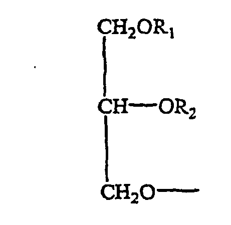

The term "N-N-dialkylamino" denotes

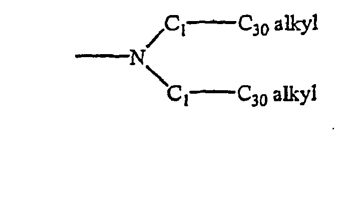

The term "1,2-diacyloxy-3-aminopropane" denotes 2-fatty acyl chains C₁-C₃₀ bonded to the 1- and 2-position of propane by an ester linkage. The acyl groups can be saturated or have varying degrees of unsaturation. The 3-position of the propane molecule has a -NH- group attached. 1,2-diacyloxy-3-aminopropanes have the following general formula:

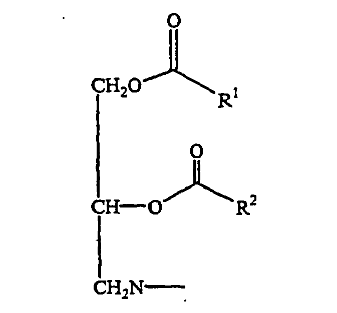

The term "1,2-dialkyl-3-aminopropane" denotes 2-alkyl chains (C₁-C₃₀) bonded to the 1- and 2-position of propane by an ether linkage. The 3-position of the propane molecule has a -NH- group attached. 1,2-dialkyl-3-aminopropanes have the following general formula:

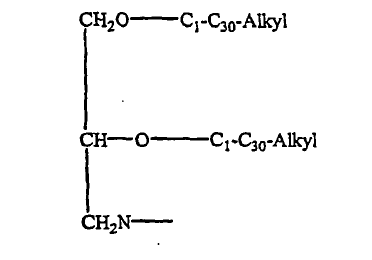

The term "non-cationic lipid" refers to any neutral lipid as described above as well as anionic lipids. Examples of anionic lipids include, but are not limited to, phosphatidylglycerol, cardiolipin, diacylphosphatidylserine, diacylphosphatidic acid, N-dodecanoyl phosphatidylethanolamines, N-succinyl phosphatidylethanolamines, N-glutarylphosphatidylethanolamines, lysophosphatidylglycerols, and other anionic modifying groups joined to neutral lipids.

The term "cationic lipid" refers to any of a number of lipid species that carry a net positive charge at a selected pH, such as physiological pH. Such lipids include, but are not limited to, N,N-dioleyl-N,N-dimethylammonium chloride ("DODAC"); N-(2,3-dioleyloxy)propyl)-N,N,N-trimethylammonium chloride ("DOTMA"); N,N-distearyl-N,N-dimethylammonium bromide ("DDAB"); N-(2,3-dioleoyloxy)propyl)-N,N,N-trimethylammonium chloride ("DOTAP"); 3 -(N-(N,N'-dimethylaminoethane)-carbamoyl)cholesterol ("DC-Chol") and N-(1,2-dimyristyloxyprop-3-yl)-N,N-dimethyl-N-hydroxyethyl ammonium bromide ("DMRIE"). Additionally, a number of commercial preparations of cationic lipids are available which can be used in the present invention. These include, for example, LIPOFECTIN^{®} (commercially available cationic liposomes comprising DOTMA and 1,2-dioleoyl-*sn*-3-phosphoethanolamine (''DOPE''), from GIBCO/BRL, Grand Island, New York, USA); LIPOFECTAMINE^{®} (commercially available cationic liposomes comprising N-(1-(2,3-dioleyloxy)propyl)-N-(2-(sperminecarboxamido)ethyl)-N,N-dimethylammonium trifluoroacetate ("DOSPA") and("DOPE"), from GIBCO/BRL); and TRANSFECTAM^{®} (commercially available cationic lipids comprising dioctadecylamidoglycyl carboxyspermine ("DOGS") in ethanol from Promega Corp., Madison, Wisconsin, USA). The following lipids are cationic and have a positive charge at below physiological pH: DODAP, DODMA, DMDMA and the like.

The term "fusogenic" refers to the ability of a liposome or other drug delivery system to fuse with membranes of a cell. The membranes can be either the plasma membrane or membranes surrounding organelles, *e.g*., endosome, nucleus, *etc.* Fusogenesis is the fusion of a liposome to such a membrane.

The term "dendrimer" includes reference to branched polymers that possess multiple generations. In dendrimers, each generation creates multiple branch points.

The term "ligand" includes any molecule, compound or device with a reactive functional group and includes lipids, amphipathic lipids, carrier compounds, bioaffinity compounds, biomaterials, biopolymers, biomedical devices, analytically detectable compounds, therapeutically active compounds, enzymes, peptides, proteins, antibodies, immune stimulators, radiolabels, fluorogens, biotin, drugs, haptens, DNA, RNA, polysaccharides, liposomes, virosomes, micelles, immunoglobulins, functional groups, targeting agents, or toxins. The foregoing list is illustrative and not intended to be exhaustive.

The term "ATTA" or "polyamide" refers to, but is not limited to, compounds disclosed in WO 99/33494 incorporated herein by reference. These compounds include a compound having the formula 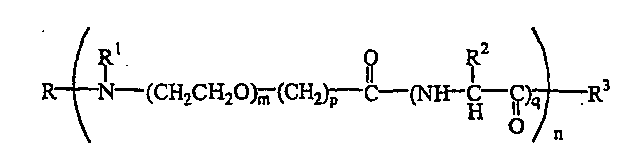 wherein: R is a member selected from the group consisting of hydrogen, alkyl and acyl; R¹ is a member selected from the group consisting of hydrogen and alkyl; or optionally, R and R¹ and the nitrogen to which they are bound form an azido moiety; R² is a member of the group selected from hydrogen, optionally substituted alkyl, optionally substituted aryl and a side chain of an amino acid; R³ is a member selected from the group consisting of hydrogen, halogen, hydroxy, alkoxy, mercapto, hydrazino, amino and NR⁴R⁵, wherein R⁴ and R⁵ are independently hydrogen or alkyl; n is 4 to 80; m is 2 to 6; p is 1 to 4; and q is 0 or 1. It will be apparent to those of skill in the art that other polyamides can be used in the compounds of the present invention.

As used herein, the term "alkyl" denotes branched or unbranched hydrocarbon chains, such as, methyl, ethyl, n-propyl, iso-propyl, n-butyl, sec-butyl, iso-butyl, tertbutyl, octa-decyl and 2-methylpentyl. These groups can be optionally substituted with one or more functional groups which are attached commonly to such chains, such as, hydroxyl, bromo, fluoro, chloro, iodo, mercapto or thio, cyano, alkylthio, heterocyclyl, aryl, heteroaryl, carboxyl, carbalkoyl, alkyl, alkenyl, nitro, amino, alkoxyl, amido, and the like to form alkyl groups such as trifluoromethyl, 3- hydroxyhexyl, 2-carboxypropyl, 2-fluoroethyl, carboxymethyl, cyanobutyl and the like.

The term "alkylene" refers to a divalent alkyl as defined above, such as methylene (-CH₂-), propylene (-CH₂CH₂CH₂-), chloroethylene (-CHClCH₂-), 2-thiobutene (-CH₂CH(SH)CH₂CH₂-), 1-bromo-3-hydroxyl-4-methylpentene (-CHBrCH₂CH(OH)CH(CH₃)CH₂-), and the like.

The term "alkenyl" denotes branched or unbranched hydrocarbon chains containing one or more carbon-carbon double bonds.

The term "alkynyl" refers to branched or unbranched hydrocarbon chains containing one or more carbon-carbon triple bonds.

The term "aryl" denotes a chain of carbon atoms which form at least one aromatic ring having preferably between about 6-14 carbon atoms, such as phenyl, naphthyl, indenyl, and the like, and which may be substituted with one or more functional groups which are attached commonly to such chains, such as hydroxyl, bromo, fluoro, chloro, iodo, mercapto or thio, cyano, cyanoamido, alkylthio, heterocycle, aryl, heteroaryl, carboxyl, carbalkoyl, alkyl, alkenyl, nitro, amino, alkoxyl, amido, and the like to form aryl groups such as biphenyl, iodobiphenyl, methoxybiphenyl, anthryl, bromophenyl, iodophenyl, chlorophenyl, hydroxyphenyl, methoxyphenyl, formylphenyl, acetylphenyl, trifluoromethylthiophenyl, trifluoromethoxyphenyl, alkylthiophenyl, trialkylammoniumphenyl, amidophenyl, thiazolylphenyl, oxazolylphenyl, imidazolylphenyl, imidazolylmethylphenyl, and the like.

The term "acyl" denotes the -C(O)R group, wherein R is alkyl or aryl as defined above, such as formyl, acetyl, propionyl, or butyryl.

The term "alkoxy" denotes -OR-, wherein R is alkyl.

The term "amido" denotes an amide linkage: -C(O)NR- (wherein R is hydrogen or alkyl).

The term "amino" denotes an amine linkage: -NR-, wherein R is hydrogen or alkyl or a terminal NH₂.

The term "carboxyl" denotes the group -C(O)O-, and the term "carbonyl" denotes the group -C(O)-.

The term "carbonate" indicates the group -OC(O)O-.

The term "carbamate" denotes the group -NHC(O)O-, and the term "urea" denotes the group -NHC(O)NH-.

The term "phosphoro" denotes the group -OP(O)(OH)O-.

The term "basic amino acid" refers to naturally-occurring amino acids as well as synthetic amino acids and/or or amino acid mimetics having a net positive charge at a selected pH, such as physiological pH. This group includes, but is not limited to, lysine, arginine, asparagine, glutamine, histidine and the like.

The term "phosphorylethanolamino" denotes the group -OP(O)(OH)OCH₂CH₂NH-.

The term "phosphorylethanolamido" denotes the group -OP(O)(OH)OCH₂CH₂NHC(O)-.

The term "phospho" denotes a pentavalent phosphorous moiety -P(O)(OH)O-.

The term "phosphoethanolamino" denotes the group -P(O)(OH)OCH₂CH₂NH-.

The term "phosphoethanolamido" denotes the group-P(O)(OH)OCH₂CH₂NHC(O)-.

The term "ethylene oxide unit" denotes the group -OCH₂CH₂-.

The term "CPL" refers to a cationic-polymer-lipid e.g., cationic-PEG-lipid. Preferred CPLs are compounds of Formula I.

The abbreviations "HBS" refers to Hepes-buffered saline, "Rho-PE" refers to rhodamine-phosphatidylethanolamine, and "LUVs" refers to "large unilamellar vesicles."

The term "array" means at least two of the nouns it modifies e.g. an array of LBSDVs is at least two LBSDVs.

The nucleic acids used in the method of the present invention can be isolated from natural sources, obtained from such sources as ATCC or GenBank libraries or prepared by synthetic methods. Synthetic nucleic acids can be prepared by a variety of solution or solid phase methods. Generally, solid phase synthesis is preferred. Detailed descriptions of the procedures for solid phase synthesis of nucleic acids by phosphite-triester, phosphotriester, and H-phosphonate chemistries are widely available. *See,* for example, Itakura, U.S. Patent No. 4,401,796; Caruthers, *et al.,* U.S. Patent Nos. 4,458,066 and 4,500,707; Beaucage, *et al., Tetrahedron Lett.,* **22**:1859-1862 (1981); Matteucci, *et al., J. Am. Chem. Soc.,* **103:**3185-3191 (1981); Caruthers, *et al., Genetic Engineering,* **4**:1-17 (1982); Jones, chapter 2, Atkinson, *et al.,* chapter 3 and Sproat, *et al.,* chapter 4 in *Oligonucleotide Synthesis: A Practical Approach,* Gait (ed.), IRL Press, Washington D.C. (1984); Froehler, *et al., Tetrahedron Lett.,* 27:469-472 (1986); Froehler, *et al., Nucleic Acids Res.,***14**:5399-5407 (1986); Sinha, *et al. Tetrahedron Lett.,* **24**:5843-5846 (1983); and Sinha, *et al., Nucl. Acids Res.,* **12**:4539-4557 (1984), all of which are incorporated herein by reference.

A "therapeutic gene" is one whose gene product performs a clinically useful function. For example, where the therapeutic gene is used to transform cancer cells, the therapeutic gene will inhibit the growth of the cancer cells. The therapeutic gene is preferably one whose gene product has low toxicity to non-target tissues, and high toxicity to the disease (*e.g.* cancer) site. For example, when delivered in the preferred lipid-nucleic acid (*e.g.,* lipid-plasmid particles) particles of the invention, the gene product preferably has greater toxicity to tumor cells than liver or spleen cells, where a large portion of particles can normally be cleared. Alternatively, a therapeutic gene may be delivered to a treatment site, which is not a disease site, but which activates an immunologic or other response which is then favorable for the amelioration of the disease or disorder being treated. Examples of therapeutic genes useful in the methods of the present invention include, but are not limited to, genes for: pro-apoptotic proteins; tumor suppressors (*e.g.,* p53, Rb1 (Retinoblastoma), *etc.*); cytokines (such as Interleukin-2, Interleukin-12, *etc.);* heat shock proteins; immunogenic antigens (such as tumor-specific proteins, *etc.*); genes activated in embryos only; Endostatin, Angiostatin, Thrombospondin, and other inhibitors of angiogenesis; Enzymes used in GDEPT combinations (*i.e.,* suicide genes used in conjunction with a non-toxic pro-drug), such as Thymidine Kinase from Herpes simplex virus (HSV-TK); cytosine deaminase; porfirin; TIMP-2 (tissue inhibitor of metallo proteinase-2); plant, bacterial or fungal toxin genes, such as saporin, ricin, diphtheria toxin, cholera toxin; viral protein genes, such as E1A; mutated E6; SV40 Tag or viral protein genes which effect plasmid maintenance and/or copy number, such as EBNA-1; transcription plasmids encoding ribozymes or antisense oligonucleotides, Adenosine Deaminase; CFTR-Cystic Fibrosis; GM-CSF, IL-4, IL-2, IL-7, IL-10; Carcineombryonic Antigen; HLA-B7; TNF; T-Cell Receptor Antibody; CEA; Ig; IFN-g; MART-1; Chimeric Antibody/TCR; Prostate Specific Antigen; anti-erbB-2; Single Chain Antibody; BRCA-1; Alpha-1 Antitrypsin; p47 phax; Fanconi Anemia Complementation Group C; Glucocerbrosidase; Iduronato-2-Sulfatase; Purine Nulceaside Phosphorylase. Other therapeutic genes are continually being discovered and can be used in the methods of the present invention. Therapeutic genes are generally delivered as part of an expression construct, although other formats are possible.

### II. LIPID-BASED SYSTEMIC DELIVERY VEHICLES ("LBSDVs")

### A. Architecture of the LBSDV

The present invention provides an in vitro method for identifying a nucleic acid-lipid particle capable of selectively targeting a nucleic acid to a heart, liver or tumor tissue site.

The nucleic acid-lipid particle comprises a nucleic acid, such as a therapeutic gene. Suitable nucleic acids include, but are not limited to, plasmids, antisense oligonucleotides, and ribozymes. Nucleic acid is negatively charged and can be combined with a positively charged entity to form a lipid complex suitable for formulation and cellular delivery. According to the present invention, a cationic lipid is included in the formulation, i.e. lipid-nucleic acid particle. By designing a lipid-based systemic delivery vehicle having a plurality of constituent parts, and thereafter varying the amounts of each of the plurality of constituent parts, it is possible to impart tissue selectivity to the LBSDV (lipid-based systemic delivery vehicles and nucleic acid-lipid particle, respectively). As used herein "tissue selectivity" means that the LBSDV has a propensity to accumulate or target a specific tissue site, and/or there is expression of a therapeutic gene at a specific tissue site. As explained in detail below, tissue selectivity can be determined and confirmed using both *in vivo* and *in vitro* methods. Once tissue selectivity has been imparted to the LBSDV, it is possible to selectively target an active agent to a specific tissue site.

In one preferred aspect, the LBSDV is a stable plasmid-lipid particle ("SPLPs"). The SPLPs or nucleic acid-lipid particles of the present invention have a plurality of constituent parts. The SPLP comprises the following constitutive parts: (a) a nucleic acid; (b) 1.0 mole % to 45 mole % of a cationic lipid; (c) 0.0 mole % to 90 mole % of another lipid, such as a non-cationic lipid; (d) 1.0 mole % to 10 mole % of a bilayer stabilizing component, (e) 0.0 mole % to 60 mole % cholesterol; and (f) 0.0 mole % to 10 mole % of cationic polymer lipid. Advantageously, as explained above, it has been discovered that by varying each of the plurality of constituent parts, it is possible to impart tissue selectivity to the SPLP.

For example, in certain indications, such as cirrhoses of the liver, a liver tumor, etc., it is desirable to target conventional drugs or nucleic acids to various tissue such as the liver. By architecturally designing the constituent parts of the LBSDV, tissue specific accumulation *e.g.*, liver accumulation *i.e*., a propensity to be directed to the liver, is imparted to the LBSDV (e.g. SPLP).

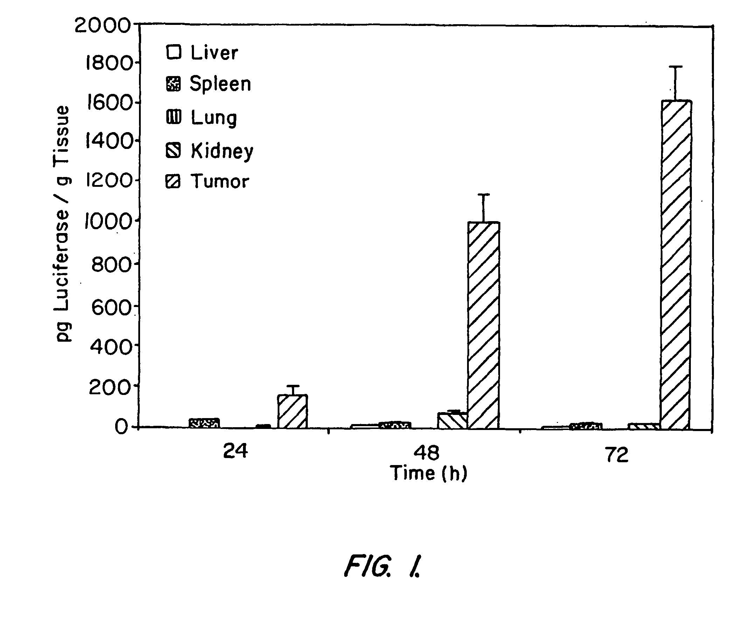

An exemplar LBSDV (*e.g.*, SPLP) designed for tumor delivery is illustrated in Figure 1. In this embodiment, the SPLP comprises DOPE, DODAC and PEGC20. Preferably, the ratio of DOPE:DODAC:PEGC20 is 82.5 mole % to 7.5 mole % to 10 mole %. Following systemic administration to Neuro-2A tumor bearing mice, the biodistribution of such SPLPs was determined. Figure 1 shows that it is possible to architecturally design the constituent parts of the LBSDV for tumor accumulation *i*.*e*., a propensity to be directed to the tumor or to have greater expression of a therapeutic gene in the tumor relative to other tissue.

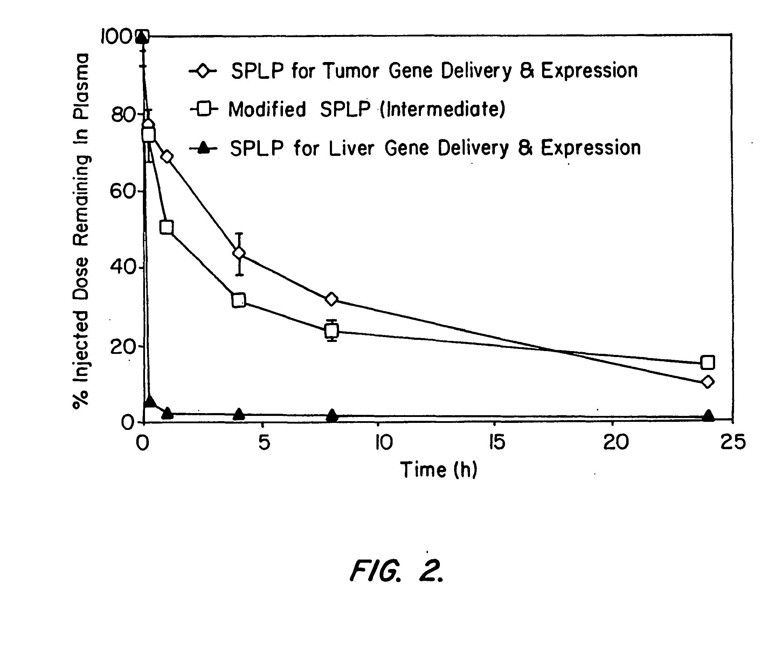

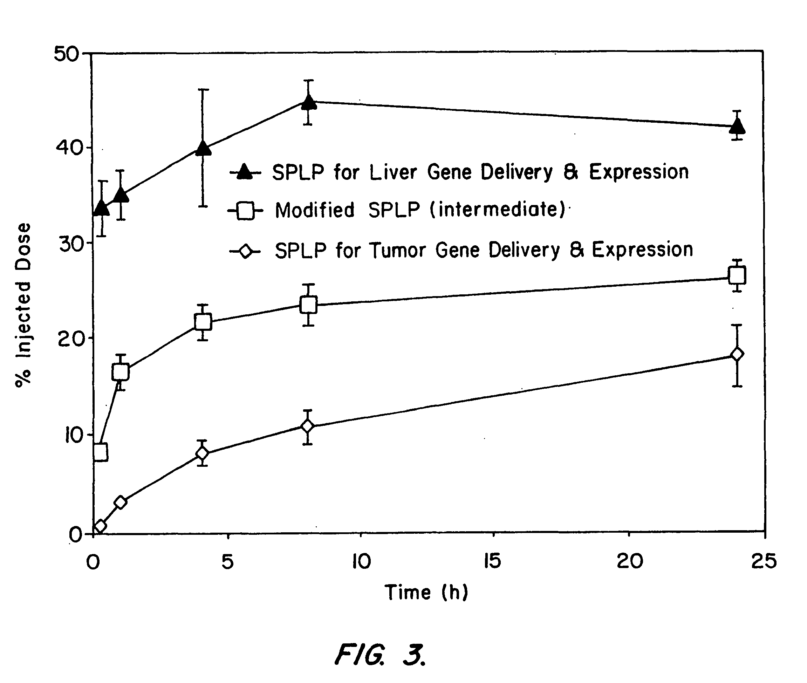

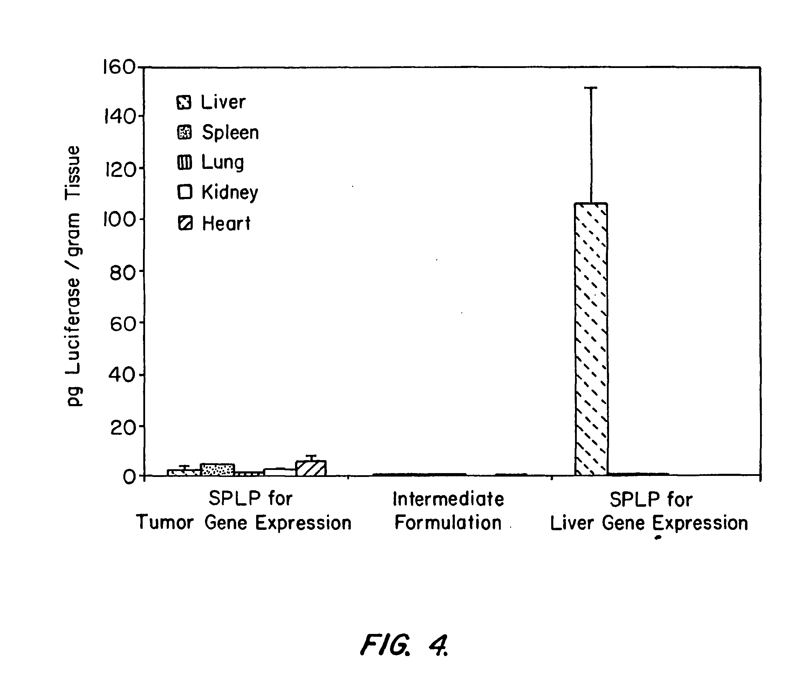

Figure 2 shows that is possible using the method of the present.invention, to engineer the SPLP to impart liver tissue selectivity. In this embodiment, the LBSDV is designed for tissue targeting, wherein the tissue site is the liver. An exemplar LBSDV (*e.g.*, SPLP) designed for liver delivery is illustrated in Figures 3-4. Figures 3 and 4 show the results of engineering the LBSVD such as SPLPs, to obtain liver selectivity, wherein the SPLP comprises DOPE, CHOL, DODAC, PEGC20, and a CPL. More particularly, the SPLP comprises DOPE:CHOL:DODAC:PEGC20:CPL in a ratio of 31 mole % to 45 mole % to 14 mole % to 10 mole % to 4 mole %.

In addition to the aforementioned tumor and liver tissues, in still yet another embodiment, it is possible to selectively deliver a LBSDV to heart tissue. For instance, using an LBSDV such as a SPLP wherein the constitutive parts comprise DOPE, DODAC and PEGC₂₀ in a ratio of 76 mole % to 14 mole % to 10 mole % selective heart delivery is achieved.

One of the constitutive parts of the LBSDVs of the present invention is a cationic lipid. As explained above, a cationic lipid generally refers to a lipid with a cationic head group situated at or near the liposome membrane (when incorporated in a liposome). CPLs, another preferred constitutive part of the LBSVDs, are distinguished from cationic lipids by the polymer "W" which in certain instances, has the effect of placing the cationic charge at a significant distance from the membrane of the LBSDV.

Examples of suitable cationic lipids include, but are not limited to, the following: N,N-dioleyl-N,N-dimethylammonium chloride (DODAC), N,N-distearyl-N,N-dimethylammonium bromide (DDAB), N-(1-(2,3-dioleoyloxy)propyl)-N,N,N trimethylammonium chloride (DOTAP), N-(1-(2,3-dioleoyloxy)propyl)-N,N-dimethylammonium chloride (DODAP), N-(1-(2,3-dioleyloxy)propyl)-N,N,N-trimethylammonium chloride (DOTMA), and N,N-dimethyl-2,3-dioleyloxy)propylamine (DODMA), and a mixture of two or more of the above. Other cationic lipids include DC-Chol, (*see,* Gao, *et al., Biochem. Biophys*. *Res. Comm.,* 179:280-285 (1991); DDAB; DMRIE; DODAC (see, WO96/10390, which is incorporated herein by reference); DOGS; DOSPA; DOTAP; and DOTMA. In a presently preferred embodiment, *N*,*N*-dioleoyl-*N*,*N-*dimethylammonium chloride is used in combination with a phosphatidylethanolamine.

In addition, other compositions containing cationic lipids useful in producing lipid-based carriers for gene and oligonucleotide delivery are LIPOFECTIN (U.S. Patents Nos. 4,897,355; 4,946,787; and 5,208,036 issued to Eppstein, *et al*.) and LIPOFECTACE (U.S. Patent No. 5,279,883 issued to Rose). Both agents, as well as other transfecting cationic lipids, are available from Life Technologies, Inc. in Gaithersburg, Maryland.

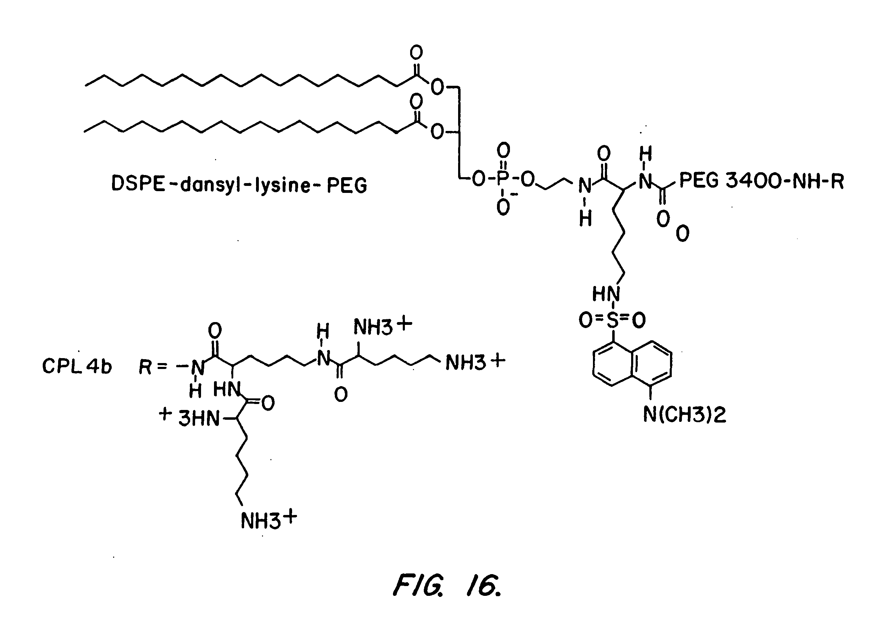

Another preferred constitutive part of the LBSDVs of the present invention is a cationic polymer lipid ("CPL", *see,* Figure 16.) In certain applications, the polymer length *i.e.,* molecular weight ("MW") of "W" in te CPL is shorter than the normal neutral PEG chains (M.W.2000-5000 Daltons) used for stealth liposomes. In such instances, the shorter polymer of the CPL is about 250 to about 3000 Daltons and more preferably, about 1000 to about 2000 Daltons. A complete description of CPLs is disclosed in U.S Patent Application No. 09/553,639, filed on April 20,2000, and PCT Publication No.WO 00/62813, published October 26,2000 which are incorporated herein by reference for all purposes.

In another embodiment, the polymer length of "W" in the CPL has a larger MW than the normal neutral PEG chains used for stealth liposomes. In such instances, the BSC is for example, a PEG₁₀₀₀-lipid, and the compound of Formula I has a formula of, for example, A- PEG₃₄₀₀-Y.

In certain formulations and applications, the type of CPL, *i.e*. the length of the polymer chain, the amount of cationic charge per molecule, and the amount of such CPL in a given formulation, *e.g*., SPLP, can be optimized to obtain the desired clearance properties. In certain instances, longer chain CPLs and higher levels of such CPLs in a given formulation result in increased transfection. In other instances, shorter chain CPLs incorporated in the formulation result in longer circulation lifetimes in animals.

Typically, the CPL is present in the LBSDVs of the present invention at a concentration ranging from about 0.05 mole percent to about 10 mole percent. In a presently preferred embodiment, the CPL is present at a concentration ranging from 0.05 mole percent to about 10 mole percent. In an even more preferred embodiment, the CPL is present at a concentration ranging from 0.05 mole percent to about 10 mole percent. One of ordinary skill in the art will appreciate that the concentration of the CPL can be varied depending on the CPL employed and the rate at which the liposome is to become fusogenic.

Another preferred constitutive part of the LBSDVs of the present invention are bilayer stabilizing components (BSC). Suitable BSCs include, but are not limited to, polyamide oligomers, peptides, proteins, detergents, lipid-derivatives, PEG-lipids such as PEG coupled to phosphatidylethanolamine, and PEG conjugated to ceramides *(see,* U.S. Patent No. 5,885,613, which is incorporated herein by reference). Preferably, the bilayer stabilizing component is a PEG-lipid, or an ATTA-lipid. As discussed herein, in certain preferred instances, the PEG or the ATTA of the BSC has a greater molecular weight compared to the polymer "W" of the CPL. In other instances, the BSC has a smaller molecular weight compared to the "W" of the polymer. The present invention encompasses all such variations.

In one embodiment, the LBSDVs of the present invention comprise: a lipid capable of adopting a non-lamellar phase, yet capable of assuming a bilayer structure in the presence of a bilayer-stabilizing component (such as a PEG-lipid derivative); and a bilayer-stabilizing component reversibly associated with the lipid to stabilize the lipid in a bilayer structure. Such fusogenic liposomes *(see,* PCT Publication WO 96/40964, published to December 19, 1996) are advantageous because the rate at which they become fusogenic can - be not only predetermined, but also varied as required over a time scale of a few minutes to several tens of hours. It has been found, for example, that by controlling the composition and concentration of the bilayer-stabilizing component, one can control the rate at which the BSC exchanges out of the liposome *in vivo* and, in turn, the rate at which the liposome becomes fusogenic *(see,* U.S. Patent No. 5,885,613). For instance, it has been found that by controlling the length of the lipid acyl chain(s), one can control the rate at which the BSC exchanges out of the liposome *in vivo* and, in turn, the rate at which the liposome becomes fusogenic. In particular, it has been discovered that shorter acyl chains (*e.g.*, C-8) exchange out of the liposome more rapidly than longer acyl chains (*e.g.*, C-20). Alternatively, by controlling the composition and concentration of the BSC, one can control the rate at which the BSC is degraded, *i*.*e*., broken down, by endogenous systems, e.g., endogenous enzymes in the serum, and, in turn, the rate at which the liposome becomes fusogenic.

As such, in one embodiment, the lipids which can be used to form the LBSVDs of the present invention are those which adopt a non-lamellar phase under physiological conditions or under specific physiological conditions, e.g., in the presence of calcium ions, but which are capable of assuming a bilayer structure in the presence of a BSC. Such lipids include, but are not limited to, phosphatidylenthanolamines, ceramides, glycolipids, or mixtures thereof. Other lipids known to those of skill in the art to adopt a non-lamellar phase under physiological conditions can also be used. Moreover, it will be readily apparent to those of skill in the art that other lipids can be induced to adopt a non-lamellar phase by various non-physiological changes including, for example, changes in pH or ion concentration (*e.g.,* in the presence of calcium ions) and, thus, they can also be used to form the fusogenic liposomes of the present invention. In a presently preferred embodiment, the LBSVD is prepared from a mixture of a phosphatidylethanolamine (saturated or unsaturated) and a cationic lipid.

In accordance with the present invention, lipids adopting a non-lamellar phase under physiological conditions can be stabilized in a bilayer structure by BSCs which are either bilayer forming themselves, or which are of a complementary dynamic shape. The non-bilayer forming lipid is stabilized in the bilayer structure only when it is associated with, *i*.*e*., in the presence of, the BSC. In selecting an appropriate BSC, it is preferable that the BSC be capable of transferring out of the liposome, or of being chemically modified by endogenous systems such that, with time, it loses its ability to stabilize the lipid in a bilayer structure. Only when liposomal stability is lost or decreased can fusion of the liposome with the plasma membrane of the target cell occur. The BSC-lipid, therefore, is "reversibly associated" with the lipid and only when it is associated with the lipid is the lipid constrained to adopt the bilayer structure under conditions where it would otherwise adopt a non-lamellar phase. As such, the BSC-lipids of the present invention are capable of stabilizing the lipid in a bilayer structure, yet they are capable of exchanging out of the liposome, or of being chemically modified by endogenous systems so that, with time, they lose their ability to stabilize the lipid in a bilayer structure, thereby allowing the liposome to become fusogenic.

Another preferred constituent part of the LBSDVs of the present invention is another lipid such as a non-cationic lipid. Non-cationic lipids include, neutral lipids and anionic lipids. Preferred non-cationic lipids include, but are not limited to, dioleoyl phosphatidylcholine (DOPE), POPC, and egg phosphatidylcholine (BPC).

In another embodiment of the present invention, the LBSDV (*e*.*g*. liposomes) optionally comprise cholesterol. It has beep determined that when cholesterol-free liposomes are used *in vivo*, they have a tendency to absorb cholesterol from the plasma lipoproteins and cell membranes. Cholesterol, if included, is generally present at a concentration ranging from 0.0 mole percent to about 60 mole percent and, more preferably, at a concentration ranging from 15 mole percent to 60 mole percent and most preferably from 35 mole percent to 45 mole percent.

### B. Preparations LBSDVs such as CPL-Liposomes

The following discussion refers generally to liposomes; however, it will be readily apparent to those of skill in the art that this same discussion is fully applicable to the LBSDVs of the present invention (lipid-nucleic acid particles, sPLPs). In a preferred method, SPLPs are made in accordance with the teachings ofPCT Publication WO 96/40964, published on December 19, 1996.

A preferred LBSDV of the present invention is a CPL-liposome. A variety of general methods for making CPL-containing SPLPs (or "CPL-liposomes'') are discussed herein. Two general techniques include "post-insertion," that is, insertion of a CPL into for example, a pre-formed liposome vesicle, and "standard" techniques, wherein the CPL is included in the lipid mixture during for example, the liposome formation steps. The post-insertion technique results in liposomes having CPLs mainly in the external face of the liposome bilayer membrane, whereas standard techniques provide liposomes having CPLs on both internal and external faces.

In particular, "post-insertion" involves forming LBSDVs (by any method), and incubating the pre-formed vesicles in the presence of CPL under appropriate conditions (usually 2-3 hours at 60°C). Between 60-80% of the CPL can be inserted into the external leaflet of the recipient vesicle, giving final concentrations up to 7 mol % (relative to total lipid). The method is especially useful for vesicles made from phospholipids (which can contain cholesterol) and also for vesicles containing PEG-lipids (such as PEG-Ceramide).

In an example of a "standard" technique, the LBSDVs of the present invention can be formed by extrusion. In this embodiment, all of the lipids including CPL, are co-dissolved in chloroform, which is then removed under nitrogen followed by high vacuum. The lipid mixture is hydrated in an appropriate buffer, and extruded through two polycarbonate filters with a pore size of 100 nm. The resulting vesicles contain CPL on both internal and external faces. In yet another standard technique, the formation of CPL-LUVs can be accomplished using a detergent dialysis or ethanol dialysis method, for example, as discussed in U.S. Patent Nos. 5,976,567 and 5,981,501, both of which are incorporated herein by reference.

### C. Use Of LBSDVs As Drug Delivery Vehicles

Nucleic acid-lipid particles are useful for the systemic or local delivery of nucleic acids to a heart, liver or tumor tissue site. Lipid-based delivery systems are described in greater detail in, for example, the following: WO95/32706, U.S. Patent Nos. 5,705,385, 5,976,567, 5,981,501, 60/055,094, 08/856,374, WO99/04819 and WO99/18933 the teachings of all of which are incorporated herein by reference.

The following discussion refers generally to liposomes; however, it will be readily apparent to those of skill in the art that this same discussion is fully applicable to the drug delivery systems of the present invention lipid-nucleic acid particles, SPLPs.

For the delivery of therapeutic agents, the LBSDVs can be loaded with a nucleic acid as therapeutic agent and administered to the subject requiring treatment.

As mentioned above, the LBSDVs can be used in the delivery of therapeutic genes or oligonucleotides intended for example, to induce or to block production of some protein within the cell. Nucleic acid is negatively charged and may be combined with a positively charged entity to form a lipid complex or a fully encapsulated stable plasmid-lipid particle.

Particularly useful antisense oligonucleotides are directed to targets such as c-myc, bcr-abl, c-myb, ICAM-1, C-erb B-2 and BCL-2. The LBSDVs of the present invention are also useful in the delivery of nucleic acids, plasmid DNA, minichromosomes and ribozymes.

In certain embodiments, it is desirable to target the LBSDVs of this invention using targeting moieties that are specific to a cell type or tissue. Targeting of the LBSDVs using a variety of targeting moieties, such as ligands, cell surface receptors, glycoproteins, vitamins (*e*.*g*., riboflavin) and monoclonal antibodies, has been previously described (*see*, *e*.*g*., U.S. Patent Nos. 4,957,773 and 4,603,044, the teachings of which are incorporated herein by reference). The targeting moieties can comprise the entire protein or fragments thereof. However, preferably the nucleic acid-lipid particle used in the method according the present invention preferably excludes targeting moieties.

In some cases, the diagnostic targeting of the LBSDVs can subsequently be used to treat the targeted cell or tissue. For example, when a toxin is coupled to a targeted liposome, the toxin can then be effective in destroying the targeted cell, such as a neoplasmic cell.

In another aspect, the present invention provides a method for increasing intracellular delivery of a LBSDV comprising: incorporating into the LBSDV a compound of Formula I, thereby increasing the intracellular delivery of the LBSDV compared to a LBSDV without a compound of Formula I. The compounds of Formula I increase intracellular delivery about 10 fold to about 1000 fold and preferably, about 10 fold to about 100,000 fold.

### D. Loading and Administering the Liposomes

The following discussion refers generally to liposomes; however, it will be readily apparent to those of skill in the art that this same discussion is fully applicable to the (lipid-nucleic SPLPs). Methods of loading conventional drugs into liposomes include, for example, an encapsulation technique, loading into the bilayer and a transmembrane potential loading method. Again, methods of incorporating nucleic acids into the LBSDVs are disclosed in PCT Publications WO 96/40964, published to December 19, 1996; WO99/05303, published February 4, 1999; and WO99/18933, published April 22, 1999.

In one encapsulation technique, the nucleic acid as drug and liposome components are dissolved in an organic solvent in which all species are miscible and concentrated t a dy film. A buffer is then added to the dried film and liposomes are formed the drug incorporated into the vesicle walls. Alternatively, the drug can be placed into a buffer and added to a dried film of only lipid components. In this manner, the drug will become encapsulated in the aqueous interior of the liposome. The buffer, which is used in the formation of the liposomes, can be any biologically compatible buffer solution of, for example, isotonic saline, phosphate buffered saline or other law ionic strength buffers. Generally, the drug will be present in an amount of from about 0.01 ng/mL to about 50 mg/mL. The resulting liposome*s* with the drug incorporated in the aqueous interior or in the membrane are then optionally sized as described above.

Transmembrane potential loading has been described in detail in U.S. Patent No. 4,885,172, U.S. Patent No. 5,059,421, and U.S. Patent No. 5,171,578, the contents of which are incorporated herein by reference. Briefly, the transmembrane potential loading method can be used with essentially any conventional drug, which can exist in a charged state when dissolved in an appropriate aqueous medium. Preferably, the drug will be relatively lipophilic so that it will partition into the liposome membranes. A transmembrane potential is created across the bilayers of the liposomes or protein-liposome complexes and the drug is loaded into the liposome by means of the transmembrane potential. The transmembrane potential is generated by creating a concentration gradient for one or more charged species *(e.g.,* Na⁺, K⁺ and/or H⁺) across the membranes. This concentration gradient is generated by producing liposomes having different internal and external media and has an associated proton gradient. Drug accumulation can than occur in a manner predicted by the Henderson-Hasselbach equation.

The liposome compositions can by administered to a subject according to standard techniques. Preferably, pharmaceutical compositions of the liposome compositions are administered parenterally, *i.e.*, intraperitoneally, intravenously, subcutaneously or intramuscularly. More preferably, the pharmaceutical compositions are administered intravenously by steady infusion. Suitable formulations are found in *Remington's Pharmaceutical Sciences,* Mack Publishing Company, Philadelphia, PA, and 17th ed. (1985). The pharmaceutical compositions can be used, for example, to diagnose a variety of conditions, or treat a diseased state. The diseases include, but are not limited to, inflammation associated with rheumatoid arthritis, post-ischemic leukocyte-mediated tissue damage (reperfusion injury), acute leukocyte-mediated lung injury (*e.g*., adult respiratory distress syndrome), septic shock, and acute and chronic inflammation, including atopic dermatitis and psoriasis. In addition, various neoplasms and tumor metastases can be treated.

Preferably, the pharmaceutical compositions are administered intravenously. Thus, this invention provides compositions for intravenous administration which comprise a solution of the liposomes suspended in an acceptable carrier, preferably an aqueous carrier. A variety of aqueous carriers can be used, e.g., water, buffered water, 0.9% isotonic saline, and the like. These compositions can be sterilized by conventional, well known sterilization techniques, or may be sterile filtered. The.resulting aqueous solutions may be packaged for use as is or lyophilized, the lyophilized preparation being combined with a sterile aqueous solution prior to administration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, *etc.*

The concentration of active ingredient in the pharmaceutical formulations can vary widely, *i*.*e*., from less than about 0.05%, usually at or at least about 2-5% to as much as 10 to 30% by weight and will be selected primarily by fluid volumes, viscosities, *etc.,* in accordance with the particular mode of administration selected. For diagnosis, the amount of composition administered will depend upon the particular label used (*i*.*e*., radiolabel, fluorescence label, and the like), the disease state being diagnosed and the judgment of the clinician.

### III. LIBRARIES OF LBSVDs SUCH AS SPLPSs

Combinatorial chemistry is a generic term that describes a series of innovative technologies that are designed to automate and simplify the selection, synthesis, and formulation of candidate LBSDVs and combinations thereof into a library. The initial step of a combinatorial process is selection of the constituent parts of compounds such as cationic lipids, non-cationic lipids and CPLs, for inclusion in a library of LBSDVs. A major focus in the formulation of the library is the automation of each step of various operations. In many cases, 96-well or 384-well microtiter plates are used for the dispensing of reagents. Automated systems are available that control temperature of the reactions, volume of reaction reagents, inertness of the reaction atmosphere, etc. In addition, highly automated sampling handling and analysis has been developed to analyze the volume of LBSDVs in the library.

Preparation of combinatorial chemical libraries is well known to those of skill in the art. Using similar methods, it is possible to prepare large libraries of LBSDVs. The array or library of the SPLPs comprise a first nucleic acid-lipid particle having a first plurality of constituent parts and a first tissue selectivity; and a second nucleic acid-lipid particle having a second plurality of constituent parts and a second tissue selectivity, wherein the first and second nucleic acid-lipid particles are constitutively different. It is noted that the constituent parts can be the same or different. For example, in one embodiment, the first SPLP comprises a nucleic acid, a cationic lipid, a BSC and cholesterol; and the second SPLP comprises a nucleic acid, a cationic lipid, a BSC, cholesterol and a CPL.

Devices for the preparation of combinatorial libraries are commercially available *(see,* e.g., 357 MPS, 390 MPS, Advanced Chem Tech, Louisville KY, Symphony, Rainin, Woburn, MA, 433A Applied Biosystems, Foster City, CA, 9050 Plus, Millipore, Bedford, MA). A number of well known robotic systems have also been developed for solution phase chemistries. These systems include automated workstations like the automated synthesis apparatus developed by Takeda Chemical Industries, LTD. (Osaka, Japan) and many robotic systems utilizing robotic arms (Zymate II, Zymark Corporation, Hopkinton, Mass.; Orca, Hewlett-Packard, Palo Alto, Calif.) which mimic the manual synthetic operations performed by a person of skill in the art. The nature and implementation of modifications to these devices (if any) so that they can operate as discussed herein will be apparent to skilled artisans. In addition, numerous combinatorial libraries are themselves commercially available (*see*, *e.g.*, ComGenex, Princeton, N.J., Asinex, Moscow, Ru Tripos, Inc., St. Louis, MO, ChemStar, Ltd, Moscow, RU, 3D Pharmaceuticals, Exton, PA, Martek Biosciences, Columbia, MD, etc.).

### IV. DETERMINING TISSUE SELECTIVITY

### A. In vitro Method

In order to determine the tissue selectively of the nucleic acid*-*lipid particle, the following *in vitro* assay may be used. The method comprises providing a multiwell plate having a first well and a second well, each well having a tissue type disposed therein. In each well is dispensed a nucleic acid-lipid particle having a first plurality of constituent parts, thereafter, the transfection efficiency of the nucleic acid of the nucleic acid-lipid particles in the desired tissue is determined.

In certain aspects, each well in the multiwell plate has the same type of tissue such as liver cells. In this aspect, each wall of the multiwell titer plate will have a different LBSVD such as a SPLP dispensed therein, *i.e.*, the plurality of constituent parts of the LBSVD such as a SPLP are varied. Alternatively, each well of the multiwell plate can have a different type of tissue disposed therein. In this aspect, the plurality of constituent parts of the LBSDV are held constant.

The tissue or cells are contacted with a nucleic acid-lipid particle for a period of sufficient time, to effectuate transfection. Thereafter, the efficiency of the transfection is determined to evaluate tissus selectivity.

### B. In vivo Method

In order to determine the tissue selectively of the nucleic acid*-*lipid particle, the following in vivo assay may be used. The assay method comprising: injecting a lipid-based systemic delivery vehicle having a plurality of constituent parts into the tail vein of a mouse to generate a biodistribution pattern; and analyzing the biodistribution pattern thereby determining tissue selectivity. In certain aspects, the mouse is an array of mice, which are administered LBSDVs such as SPLPs with radioactive agents contained therein. After administration, biodistribution studies are carried out. Example 1 set forth below illustrates such a study. The accumulation of the radioactive agent, such as the accumulation of [³H]CHE in organs and tumors is determined. Alternatively, the active agent is a gene encoding an enzyme such as luciferase. Thereafter, the propensity of the LBSDV to deliver a certain agent to the target site can be determined.

### V. DETERMINING NUCLEIC ACID FUNCTIONALITY

After tissue selectivity has been imparted into a LBSDV; it is possible to ascertain nucleic acid functionality with a method comprising: administering a lipid-based systemic delivery vehicle having tissue selectivity to a mammal, the lipid-based systemic delivery vehicle comprising a nucleic acid; and analyzing the effect if any, of the nucleic acid on a specific tissue site, thereby determining nucleic acid functionality. Nucleic acids of all types may be associated with the LBSDVs of the present invention and thus can be subsequently delivered to a specific tissue site. These include DNA, RNA, DNA/RNA hybrids (each of which may be single or double stranded), including oligonucleotides such as antisense oligonucleotides, chimeric DNA-RNA polymers, and ribozymes, those having known and unknown functionality, as well as modified versions of these nucleic acids wherein the modification may be in the base, the sugar moiety, the phosphate linkage, or in any combination thereof. Such compositions may be used to direct nucleic acid of unknown function to a specific tissue site.

From the foregoing it will be clear to those skilled in the art that the LBSVDs are useful for both *in vitro* and *in vivo* application. Using the method of the present invention, it is possible identify a nucleic acid-lipid particle capable of selectively targering a nucleic acid to a heart, liver or tumor tissue site and to analyze the effect of the nucleic acid on a specific tissue site, *i.e.*, to determining nucleic acid functionality, *e.g.*, the process of recombinant production of a protein in the liver. After the delivery of the nucleic acid to the specific site, it is possible to test compounds, which modulate the nucleic acid functionality.

In certain aspects, the nucleic acids comprises an essential gene or fragment thereof, in which the target tissue cell or cells is deficient in some manner. This can occur where the gene is lacking or where the gene is mutated resulting in under- or over-expression. The nucleic acids can also comprise antisense oligonucleotides. Such antisense oligonucleotides can be constructed to inhibit expression of a target gene. The foregoing are examples of nucleic acids that may be used with the present invention, and should not be construed to limit the invention in any way. Those skilled in the art will appreciate that other nucleic acids will be suitable for use in the present invention as well.

The following examples serve to illustrate, but not to limit the invention.

### EXAMPLES

### Materials

Six large-scale luciferase plasmid TCS formulations were prepared. All were labeled with [3-H]-CHE to facilitate PK and biodistribution studies. They are as follows:

| | | |
|---|---|---|
| A: 303 | DOPE:DODAC:PEGC20::82.5:7.5:10 | 4(lead systemic SPLP) |
| B: 303 + CPL | 303 w/ 4 mol % CPL-4-1k | |
| C: 314 | DOPE:DODAC:PEGC20::76:14:10 | (high cationic lipid) |
| D: 314 + CPL | 314 w/ 4 mol % CPL-4-1k | |
| E:314C | DOPE:CHOL:DODAC:PEGC20::31:45:14:10 | (cholesterol) |
| F: 314C + CPL | 314C w/ 4 mol % CPL-4-1k | |

These formulations were prepared to support *in vivo* studies.

### EXAMPLE 1

This Example illustrates the pharmacokinetics and biodistribution of SPLPs following systemic administration to Neuro-2A tumor bearing mice.

Biodistribution and gene expression were examined in parallel to differentiate between effects on gene delivery and transfection. The tumor gene expression study was extended to examine the gene expression resulting from systemic administration of PFV and the effect of calcium pre-incubation on gene expression *in vivo.*

### Test Samples/Drugs:

All samples are filtered sterilized prior to dilution to working concentration.

All samples are provided in sterile crimp top vials.

All vials are labeled with the formulation date, lipid composition, specific activity and DNA concentration.

All samples are provided at 0.5 mg/ml DNA.

3[H]CHE is incorporated at 1 µCi/mg Lipid.

| Sample | Treatment | Contents |
|---|---|---|
| A: | [3-H]CHE-SPLP | SPLP |
| B: | [3-H]CHE-SPLP-CPL | SPLP w/ CPL-4-1k |
| C: | [3-H]CHE-HCL-SPLP | SPLP w/ high cationic lipid |
| D: | [3-H]CHE-HCL-SPLP-CPL | SPLP w/ high cationic lipid and CPL-4-1k |
| E: | [3-H]CHE-HCL-CHOL-SPLP | SPLP w/ high cationic lipid and cholesterol |
| F: | [3-H]CHE-HCL-CHOL-SPLP-CPL | SPLP w/ high cationic lipid, cholesterol and CPL-4-1k |

### I. Experimental Design

| | |
|---|---|
| Animals: | 140 A/J mice, female, 18-23 g; |
| Experimental Groups: | animals housed in cages of 4; |
| | 4 animals per group; |
| | 30 groups; |
| | 120 experimental mice total, 140 to be seeded. |

| **Group** | **Mice** | **Cells** | **Treatment** | **Timepoint** | **Assay** |
|---|---|---|---|---|---|
| A | 4 | Neuro2A | [3-H]CHE-SPLP | 15 min | PK/BD |
| B | 4 | Neuro2A | [3-H]CHE-SPLP | 1 hr | PK/BD |
| C | 4 | Neuro2A | [3-H]CHE-SPLP | 4 hr | PK/BD |
| D | 4 | Neuro2A | [3-H]CHE-SPLP | 8 hr | PK/BD |
| E | 4 | Neuro2A | [3-H]CHE-SPLP | 24 hr | PK/BD |
| F | 4 | Neuro2A | [3-H]CHE-SPLP-CPL | 15 min | PK/BD |
| G | 4 | Neuro2A | [3-H]CHE-SPLP-CPL | 1 hr | PK/BD |
| H | 4 | Neuro2A | [3-H]CHE-SPLP-CPL | 4 hr | PK/BD |
| I | 4 | Neuro2A | [3-H]CHE-SPLP-CPL | 8 hr | PK/BD |
| J | 4 | Neuro2A | [3-H]CHE-SPLP-CPL | 24 hr | PK/BD |
| K | 4 | Neuro2A | [3-H]CHE-HCL-SPLP | 15 min | PK/BD |
| L | 4 | Neuro2A | [3-H]CHE-HCL-SPLP | 1 hr | PK/BD |
| M | 4 | Neuro2A | [3-H]CHE-HCL-SPLP | 4 hr | PK/BD |
| N | 4 | Neuro2A | [3-H]CHE-HCL-SPLP | 8 hr | PK/BD |
| O | 4 | Neuro2A | [3-H]CHE-HCL-SPLP | 24 hr | PK/BD |
| P | 4 | Neuro2A | [3-H]CHE-HCL-SPLP-CPL | 15 min | PK/BD |
| Q | 4 | Neuro2A | [3-H]CHE-HCL-SPLP-CPL | 1 hr | PK/BD |
| R | 4 | Neuro2A | [3-H]CHE-HCL-SPLP-CPL | 4 hr | PK/BD |
| S | 4 | Neuro2A | [3-H]CHE-HCL-SPLP-CPL | 8 hr | PK/BD |
| T | 4 | Neuro2A | [3-H]CHE-HCL-SPLP-CPL | 24 hr | PK/BD |
| U | 4 | Neuro2A | [3-H]CHE-HCL-CHOL-SPLP | 15 min | PK/BD |
| V | 4 | Neuro2A | [3-H]CHE HCL-CHOL-SPLP | 1 hr | PK/BD |
| W | 4 | Neuro2A | [3-H]CHE-HCL-CHOL-SPLP | 4 hr | PK/BD |
| X | 4 | Neuro2A | [3-H]CHE-HCL-CHOL-SPLP | 8 hr | PK/BD |
| Y | 4 | Neuro2A | [3-H]CHE-HCL-CHOL-SPLP | 24 hr | PK/BD |
| Z | 4 | Neuro2A | [3-H]CHE-HCL-CHOL-SPLP-CPL | 15 min | PK/BD |
| AA | 4 | Neuro2A | [3-H]CHE-HCL-CHOL-SPLP-CPL | 1 hr | PK/BD |
| BB | 4 | Neuro2A | [3-H]CHE-HCL-CHOL-SPLP-CPL | 4 hr | PK/BD |
| CC | 4 | Neuro2A | [3-H]CHE-HCL-CHOL-SPLP-CPL | 8 hr | PK/BD |
| DD | 4 | Neuro2A | [3-H]CHE-HCL-CHOL-SPLP-CPL | 24 hr | PK/BD |

### II. Procedures

| | |
|---|---|
| Tumor Inoculum: | Cells are passaged *in vitro* as per ATCC. On day 0 of the study, cells are harvested, washed, counted and 1.5 x 10⁶ cells are injected subcutaneously in the hind flank of each mouse (injection volume: 50 µl). |
| Treatment: | Mice are treated with [3-H]CHE-SPLP (100 µg DNA) formulations administered I.V. in a total volume of 200 µl. All treatments are administered once tumor volumes are an appropriate size (approximately day 14). Animals with irregular tumors are removed from the study, Mice receive one treatment only. |
| Endpoints: | At the appropriate time-points mice are weighed, sacrificed, blood is collected by cardiac puncture and weighed, organs (liver, lung, spleen, kidney) and tumor are collected into tared fast-prep tubes and evaluated for [3-H]CHE. |
| | Formulation and tumor burden are well tolerated however mice exhibiting signs of distress associated with either the treatment or tumor burden are terminated. |
| Data Analysis: | 1) Clearance of [3-H]CHE from blood is determined. |
| | 2) Accumulation of [3-H]CHE in organs and tumor is determined. |

### III. Results

| | |
|---|---|
| Figure 2 | shows SPLP for liver gene expression. As shown therein, the SPLPs are rapidly cleared from the blood compartment. |
| Figure 3 | shows SPLP for liver gene expression. These SPLPs have been designed for effective delivery to the liver. |
| Figure 9 | shows percent injected dose in lungs of male A/J mice following a single intravenous administration of 303, 314, and 314C with or without 4 mol% CPL4-1k. |
| Figure 10 | shows percent injected dose per Neuro-2a tumor of male A/J mice following a single intravenous administration of 303, 314, and 314C with or without 4 mol% CPL4-1k. |
| Figure 11 | shows percent injected does remaining in plasma of male A/J mice following a single intravenous administration of 303, 314, and 314C with or without 4 mol% CPL4-1k |
| Figure 12 | shows percent injected dose per liver of male A/J mice following a single intravenous administration of 303, 314, and 314C with or without 4 mol% CPL4-1k |
| Figure 13 | shows percent injected dose per spleen of male A/J mice following a single intravenous administration of 303, 314, and 314C with or without 4 mol% CPL4-1k |
| Figure 14 | shows percent injected dose per in kidneys of male A/J mice following a single intravenous administration of 303, 314, and 314C with or without 4 mol% CPL4-1k |
| Figure 15 | shows percent injected does in hearts of male A/J mice following a single intravenous administration of 303, 314, and 314C with or without 4 mol% CPL4-1k |

### EXAMPLE 2

This Example illustrates luciferase gene expression following systemic administration of SPLP in Neuro2A tumor bearing mice.

### Test Samples/Drugs

All samples are filtered sterilized prior to dilution to working concentration.

All samples are provided in sterile crimp top vials.

All vials are labeled with the formulation date, lipid composition, specific activity and DNA concentration.

All samples are provided at 0.5 mg/ml DNA.

3[H]CHE is incorporated at 1 µCi/mg Lipid.

| Sample | Treatment | Contents |
|---|---|---|
| A: | [3-H]CHE-SPLP | SPLP |
| B: | [3-H]CHE-SPLP-CPL | SPLP w/ CPL-4-1k |
| C: | [3-H]CHE-HCL-SPLP | SPLP w/ high cationic lipid |
| D: | [3-H]CHE-HCL-SPLP-CPL | SPLP w/ high cationic lipid and CPL-4-1k |
| E: | [3-H]CHE-HCL-CHOL-SPLP | SPLP w/ high cationic lipid and cholesterol |
| F: | [3-H]CHE-HCL-CHOL-SPLP-CPL | SPLP w/ high cationic lipid, cholesterol and |
| | | CPL-4-1k |

### I. Experimental Design

| | |
|---|---|
| Animals: . | 140 A/J mice, female, 18-23 g; |
| Experimental Groups: | animals housed in cages of 4; |
| | 4 animals per group; |
| | 21 groups; |
| | 84 experimental mice total, 100 seeded. |

| **Group** | **Mice** | **Cells** | **Treatment** | **Timepoint** | **Assay** |
|---|---|---|---|---|---|
| A | 4 | Neuro2A | [3-H]CHE-SPLP | 24 hr | Luciferase |
| B | 4 | Neuro2A | [3-H]CHE-SPLP | 48 hr | Luciferase |
| C | 4 | Neuro2A | [3-H]CHE-SPLP | 72 hr | Luciferase |
| D | 4 | Neuro2A | [3-H]CHE-SPLP-CPL | 24 hr | Luciferase |
| E | 4 | Neuro2A | [3-H]CHE-SPLP-CPL | 48 hr | Luciferase |
| F | 4 | Neuro2A | [3-H]CHE-SPLP-CPL | 72 hr | Luciferase |
| G | 4 | Neuro2A | [3-H]CHE-HCL-SPLP | 24 hr | Luciferase |
| H | 4 | Neuro2A | [3-H]CHE-HCL-SPLP | 48 hr | Luciferase |
| I | 4 | Neuro2A | [3-H]CHE-HCL-SPLP | 72 hr | Luciferase |
| J | 4 | Neuro2A | [3-H]CHE-HCL-SPLP-CPL | 24 hr | Luciferase |
| K | 4 | Neuro2A | [3-H]CHE-HCL-SPLP-CPL | 48 hr | Luciferase |
| L | 4 | Neuro2A | [3-H]CHE-HCL-SPLP-CPL | 72 hr | Luciferase |
| M | 4 | Neuro2A | [3-H]CHE-HCL-CHOL-SPLP | 24 hr | Luciferase |
| N | 4 | Neuro2A | [3-H]CHE-HCL-CHOL-SPLP | 48 hr | Luciferase |
| O | 4 | Neuro2A | [3-H]CHE-HCL-CHOL-SPLP | 72 hr | Luciferase |
| P | 4 | Neuro2A | [3-H]CHE-HCL-CHOL-SPLP-CPL | 24 hr | Luciferase |
| Q | 4 | Neuro2A | [3-H]CHE-HCL-CHOL-SPLP-CPL | 48 hr | Luciferase |
| R | 4 | Neuro2A | [3-H]CHE-HCL-CHOL-SPLP-CPL | 72 hr | Luciferase |
| S | 4 | Neuro2A | PFV | 24 h | Luciferase |
| T | 4 | Neuro2A | PFV | 48 hr | Luciferase |
| U | 4 | Neuro2A | PFV . | 72 hr | Luciferase |

### II. Procedures

| | |
|---|---|
| Tumor Inoculum: | Cells are passaged *in vitro* as per ATCC. On day 0 of the study, cells are harvested, washed, counted and 1.5 x 10^6 cells will be injected subcutaneously in the hind flank of each mouse (injection volume: 50 µl). |
| Treatment: | Mice are treated with [3-H]CHE-SPLP (100 µg DNA) formulations administered I.V. in a total volume of 200 µl. All treatments are administered once tumor volumes are an appropriate size (starting approximately day 12). Animals with irregular tumors are removed from the study. Mice receive one treatment only. |
| Endpoints: | At the appropriate time-points mice are sacrificed, organs (liver, lung, spleen, kidney) and tumor are collected into tared fast-prep tubes and evaluated for Luciferase gene expression. Formulation and tumor burden are expected to be well tolerated however mice exhibiting signs of distress associated with either the treatment or tumor burden will be terminated at the discretion of vivarium staff. |
| Data Analysis: | 1) Luciferase gene expression. |

### III. Results

| | |
|---|---|
| Figure 1 | shows SPLP can be engineered to elicit tumor specific gene expression. |
| Figure 4 | shows gene expression in subcutaneous Neuro2A tumors following I.V. injection of SPLP. |
| Figure 5 | shows luciferase gene expression in the liver from Neuro-2a tumor bearing male A/J mice. |
| Figure 6 | shows luciferase gene expression in the heart from Neuro-2a tumor bearing male A/J mice. |
| Figure 7 | shows luciferase gene expression in Neuro-2a tumor bearing mice following a single IV administration of INEX 303. |
| Figure 8 | shows potency of SPLP: tumor gene expression following intravenous administration of SPLP. |
| Figure 17 | illustrates CPL enhance *in vitro* transfection as well. |

## Claims

1. An *in vitro* method for identifying a nucleic acid-lipid particle capable of selectively targeting a nucleic acid to a heart, liver, or tumor tissue site, said method comprising:
designing a nucleic acid-lipid particle comprising:
(a) a nucleic acid;
(b) 1.0 mole % to 45 mole % of a cationic lipid;
(c) 0,0 mole % to 90 mole % of another lipid;
(d) 1,0 mole % to 10 mole % of a bilayer stabilizing component;
(e) 0,0 mole % to 60 mole % cholesterol; and
(f) 0,0 mole % to 10 mole % of cationic polymer lipid;
and
varying the amount of each of said cationic lipid, bilayer stabilizing component, another lipid, cholesterol, and cationic polymer lipid to impart tissue selectivity for said heart, liver, or tumor tissue site, thereby identifying a nucleic acid-lipid particle capable of selectively targeting a nucleic acid to said heart, liver, or tumor tissue site.

2. The method for identifying a nucleic acid-lipid particle of claim 1, wherein said nucleic acid is selected from the group consisting of a plasmid, and antisense oligonucleotide, and a ribozyme.

3. The method for identifying a nucleic acid-lipid particle of claim 1, wherein said nucleic acid-lipid particle excludes targeting moieties.

4. The method for identifying a nucleic acid-lipid particle of claim 1, wherein said nucleic acid-lipid particle comprises DOPE, DODAC and PEGC₂₀.

5. The method for identifying a nucleic acid-lipid particle of claim 4, wherein said ratio of DOPE:DODAC:PEGC₂₀ is 76 mole % to 14 mole % to 10 mole %.

6. The method for identifying a nucleic acid-lipid particle of claim 1, wherein said nucleic acid-lipid particle comprises DOPE, CHOL, DODAC, PEGC₂₀, and a CPL.

7. The method for identifying a nucleic acid-lipid particle of claim 6, wherein the ratio of DOPE:CHOL:DODAC:PEGC₂₀:CPL is 31 mole % to 45 mole % to 14 mole % to 10 mole % to 4 mole %.

8. The method for identifying a nucleic acid-lipid particle of claim 4, wherein the ratio of DOPE:DODAC:PEGC₂₀ is 82,5 mole % to 7,5 mole % to 10 mole %.

## Patentansprüche

1. *In vitro*-Verfahren zum Identifizieren eines Nucleinsäure-Lipid-Partikels, das in der Lage ist, eine Nucleinsäure selektiv zu einer Herz-, Leber- oder Tumor-Gewebestelle zielgerichtet hinzusteuern, wobei das Verfahren umfasst:
Entwerfen eines Nucleinsäure-Lipid-Partikels, umfassend:
(a) eine Nucleinsäure;
(b) 1,0 Mol% bis 45 Mol-% eines kationischen Lipids;
(c) 0,0 Mol-% bis 90 Mol-% eines anderen Lipids;
(d) 1,0 Mol-% bis 10 Mol-% einer Bilayer-stabilisierenden Komponente;
(e) 0,0 Mol-% bis 60 Mol-% Cholesterin; und
(f) 0,0 Mol-% bis 10 Mol-% eines kationischen Polymer-Lipids;
und
Variieren der Menge jeweils des kationischen Lipids, der Bilayer-stabilisierenden Komponente, des anderen Lipids, des Cholesterin und des kationischen Polymer-Lipids, um eine Gewebeselektivität für die Herz-, Leber- oder Tumor-Gewebestelle zu verleihen, wodurch ein Nucleinsäure-Lipid-Partikel identifiziert wird, das in der Lage ist, eine Nucleinsäure selektiv zu der Herz-, Leber- oder Tumor-Gewebestelle zielgerichtet hinzusteuern.

2. Verfahren zum Identifizieren eines Nucleinsäure-Lipid-Partikels nach Anspruch 1, wobei die Nucleinsäure aus der Gruppe ausgewählt ist, die aus einem Plasmid und Antisense-Oligonucleotid und einem Ribozym besteht.

3. Verfahren zum Identifizieren eines Nucleinsäure-lipid-Partikels nach Anspruch 1, wobei das Nucleinsäure-lipid-Partikel Einheiten zum zielgerichteten Hinsteuern (_{"}targeting moieties") ausschließt.

4. Verfahren zum Identifizieren eines Nucleinsäure-Lipid-Partikels nach Anspruch 1, wobei das Nucleinsäure-Lipid-Partikel DOPE, DODAC und PEGC₂₀ umfasst.

5. Verfahren zum Identifizieren eines Nucleinsäure-Lipid-Partikels nach Anspruch 4, wobei das Verhältnis von DOPE : DODAC : PEGC₂₀ 76 Mol-% zu 14 Mol% zu 10 Mol-% beträgt.

6. Verfahren zum Identifizieren eines Nucleinsäure-Lipid-Partikels nach Anspruch 1, wobei das Nucleinsäure-Lipid-Partikel DOPE, CHOL, DODAC, PEGC₂₀ und ein CPL umfasst.

7. Verfahren zum Identifizieren eines Nucleinsäure-Lipid-Partikels nach Anspruch 6, wobei das Verhältnis von DOPE : CHOL : DODAC : PEGC₂₀ : CPL 31 Mol-% zu 45 Mol-% zu 14 Mol-% zu 10 Mol-% zu 4 Mol-% beträgt.

8. Verfahren zum Identifizieren eines Nucleinsäure-Lipid-Partikels nach Anspruch 4, wobei das Verhältnis von DOPE : DODAC : PEGC₂₀ 82,5 Mol-% zu 7,5 Mol-% zu 10 Mol-% beträgt.

## Revendications

1. Une méthode *in vitro* pour l'identification d'une particule acide nucléique - lipide ayant la capacité sélective de cibler un acide nucléique dans un site au niveau du coeur, du foie, ou d'un tissu tumoral, cette méthode comprenant :
la conception d'une particule acide nucléique - lipide comprenant :
(a) un acide nucléique ;
(b) de 1,0 mole % à 45 moles % d'un lipide cationique ;
(c) de 0,0 mole % à 90 moles % d'un autre lipide;
(d) de 1,0 mole % à 10 moles % d'un composé stabilisant en double couche
(e) de 0,0 mole % à 60 moles % de cholestérol ; et
(f) de 0,0 mole % à 10 moles % d'un polymère d'un lipide cationique
et
d'une quantité variable de chacun des éléments ; lipide cationique, composé stabilisant en double couche, autre lipide, cholestérol, et polymère de lipide cationique afin d'obtenir une caractéristique sélective de tissu d'un site cardiaque, hépatique, ou tumoral.

2. La méthode pour l'identification d'une particule acide nucléique - lipide selon la revendication 1, dans laquelle cet acide nucléique est choisi parmi le groupe composant d'un plasmide, et d'un oligonucléotide antisens, et d'un ribozyme.

3. La méthode pour l'identification d'une particule acide nucléique - lipide selon la revendication 1, dans laquelle cette particule acide nucléique - lipide exclue les groupements caractéristiques ciblant.

4. La méthode pour l'identification d'une particule acide nucléique - lipide selon la revendication 1, dans laquelle cette particule acide nucléique - lipide comprend DOPE, DODAC et PEGC₂₀.

5. La méthode pour l'identification d'une particule acide nucléique - lipide selon la revendication 4, dans laquelle le ratio de DOPE :DODAC :PEGC₂₀ est de 76 moles % pour 14 moles % pour 10 moles %.

6. La méthode pour l'identification d'une particule acide nucléique - lipide selon la revendication 1, dans laquelle cette particule acide nucléique - lipide comprend DOPE, CHOL, DODAC, PEGC₂₀ et un CPL.

7. La méthode pour l'identification d'une particule acide nucléique - lipide selon la revendication 6, dans laquelle le ratio de DOPE :CHOL :DODAC :PEGC₂₀:CPL est de 31 moles % pour 45 moles % pour 14 moles % pour 10 moles % pour 4 moles %.

8. La méthode pour l'identification d'une particule acide nucléique - lipide selon la revendication 4, dans laquelle le ratio de DOPE :DODAC :PEGC₂₀ est de 82,5 moles % pour 7,5 moles % pour 10 moles %.
